# EUROPEAN PATENT SPECIFICATION

(11) **EP 2 417 099 B1**
(45) Date of publication and mention of the grant of the patent: **23.12.2015**
(21) Application number: 10714460.2
(22) Date of filing: 06.04.2010
(51) Int. Cl.: C07C 239/20, C07D 307/82

(54) **NEW PROCESS FOR PREPARING HYDROXYLAMINES AND MEDICAMENTS**
NEUES VERFAHREN ZUR HERSTELLUNG VON HYDROXYLAMINEN UND MEDIKAMENTEN
NOUVEAU PROCÉDÉ DE PRÉPARATION D'HYDROXYLAMINES ET MÉDICAMENTS

(30) Priority: 08.04.2009 US 202813 P
(43) Date of publication of application: 15.02.2012
(73) Proprietor: Cambrex Karlskoga AB, 691 85 Karlskoga (SE)
(72) Inventor: HANSSON, Lars, O., S-691 85 Karlskoga (SE); BERGH, Anders, S-691 85 Karlskoga (SE); EKLUND, Lars, S-691 85 Karlskoga (SE)
(74) Representative: Potter Clarkson LLP
(86) International application number: PCT/GB2010/000709
(87) International publication number: WO 2010/116140

(56) References cited:
- WO-A1-2010/038029
- WO-A2-2007/140989
- WO-A2-2009/044143
- JP-A- 60 169 446
- US-A- 5 684 200
- CASTELLINO, RAPOPORT: "Synthesis of phenoxyamines" JOURNAL OF ORGANIC CHEMISTRY, vol. 49, 1984, pages 1348-1352, XP002600302
- TAKEDA, MIYATA, NAITO: "Efficient synthesis of benzofurans utilizing [3,3]-sigmatropic rearrangement triggered by N-trifluoroacetylation of oxime ethers: Short synthesis of natural 2-arylbenzofurans." EUROPEAN JOURNAL OF ORGANIC CHEMISTRY, 2007, pages 1491-1509, XP002600303
- SHERADSKY T: "O-(2,4-dinitrophenyl) oximes. Synthesis and cyclization to 5,7-dinitrobenzofurans", JOURNAL OF HETEROCYCLIC CHEMISTRY, WILEY-BLACKWELL PUBLISHING, INC, US, vol. 4, no. 3, 1 September 1967 (1967-09-01), pages 413-414, XP009165228, ISSN: 0022-152X, DOI: 10.1002/JHET.5570040322 [retrieved on 2009-03-12]

## Description

The present invention relates to a process for the preparation of benzofuran compounds, wherein the process comprises the preparation of an aromatic hydroxylamine intermediate. The process is suitable for the synthesis of compounds, e.g. drugs, for instance anti-arrhythmia drugs such as Dronedarone (N-{2-(n-butyl)-3-[4-(3-dibutylamino-propoxy)-benzoyl]-benzofuran-5-yl}methanesulfonamide).

Dronedarone is a Class III anti-arrhythmia drug for the prevention of cardiac arrhythmias such as atrial fibrillation (AF). AF is a condition characterised by an irregular heart beat and occurs when the atria (the upper chambers of the heart) contract very rapidly. This causes the lower chambers of the heart, the ventricles, to contract chaotically so that blood is inefficiently pumped to the body which can lead to tissue damage and even death.

Dronedarone is prepared *via* a stepwise procedure which involves the synthesis of a number of intermediates, including 2-butyl-3-(4-methoxybenzoyl)-5-nitrobenzofuran and 2-butyl-3-(4-hydroxybenzoyl)-5-nitrobenzofuran.

2-Butyl-3-aroyl-5-nitrobenzofurans are typically synthesised *via* Friedel-Craft acylation of 3-unsubstituted 2-butyl-5-nitrobenzofurans. Such reactions are described in e.g. Japanese patent document JP 2002-371076 and international patent application WO 2007/140989. The benzofuran-forming reactions disclosed in these applications normally proceed *via* an aromatic hydroxyimine (which is itself prepared by reaction of a hydroxyimine with an aromatic fluoride by an aromatic nucleophilic substitution reaction).

Further, international patent application WO 2009/044143 also discloses a benzofuran-forming reaction, which proceeds *via* an aromatic hydroxyimine. In this case, the aromatic hydroxyimine is prepared by reaction of an aromatic hydroxylamine with a ketone. This aromatic hydroxylamine is prepared by deprotection of a corresponding protected derivative in the presence of an acid in an organic solvent (acetonitrile).

US patent application US 3,686,237 discloses the synthesis of an aromatic hydroxylamine by reaction of an aromatic fluoride with hydroxylamine by a nucleophilic aromatic substitution reaction. There is no disclosure of a protected aromatic hydroxylamine (e.g. an imino-protected derivative).

Journal article by Castellino et al, J. Org. Chem. 1984, 49, 1348-1352 discloses the synthesis of various aromatic hydroxylamines (phenoxyamines) by an amine exchange reaction, involving an aromatic alcohol (e.g. phenol) and an appropriate amine (e.g. 2,4-dinitrophenoxyamine). It also discloses the reaction of a phenoxyamine by a nucleophilic aromatic substitution reaction of *N-*hydroxyacetimidate with an aromatic halide, followed by deprotection of the protected phenoxyamine so formed, by reaction in the presence of perchloric acid (HClO₄).

Journal article by Sheradsky et al, Tetrahedron, Vol. 28, pp 3833-3843 discloses the synthesis of various aromatic hydroxylamines, which may be prepared by reaction of the corresponding *t*-Boc protected aromatic hydroxylamine, which is deprotected by reaction in the presence of trifluoroacetic.

Journal article by Endo et al, J. Am. Chem. Soc., 1982, 104, 6393-6397, discloses the preparation of aromatic hydroxylamines, which proceeds *via* deprotection of a protected aromatic hydroxylamine (e.g. an acetyl protected derivative), by reaction in the presence of trifluoromethanesulfonic acid in dioxane.

There is a need for alternative and/or improved reactions for the formation of aromatic hydroxylamines, which may be useful intermediates in the synthesis or larger molecules. Particularly useful are processes that are viable on a commercial scale, and are suitable from an environmental stand-point, both of which are important.

The listing or discussion of an apparently prior-published document in this specification should not necessarily be taken as an acknowledgement that the document is part of the state of the art or common general knowledge.

The present invention relates to a process for the preparation of a compound of formula I, as defined in the attached claims.

Described herein is a process (which does not fall under the scope of the claims) for the preparation of a compound of formula II, 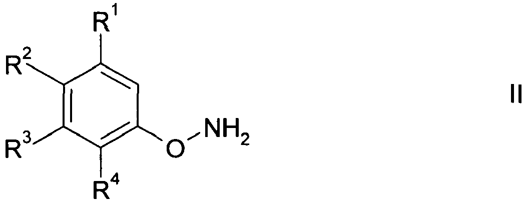 wherein:
R¹, R², R³ and R⁴ independently represent hydrogen, halo, -NO₂, -CN, -C(O)₂R^{x1}, -OR^{x2}, -SR^{x3}, -S(O)R^{x4}, -S(O)₂R^{x5}, -N(R^{x6})R^{x7}, -N(R^{x8})C(O)R^{x9}, -N(R^{x10})S(O)₂R^{x11} or R^{x12};
R^{x1}, R^{x2}, R^{x3}, R^{x6}, R^{x7}, R^{x8}, R^{x9} and R^{x10} independently represent hydrogen or C₁₋₆ alkyl optionally substituted by one or more halo atoms;
R^{x4}, R^{x5}, R^{x11} and R^{x12} independently represent C₁₋₆ alkyl optionally substituted by one or more halo atoms;
which process comprises deprotection of a compound of formula IIA, 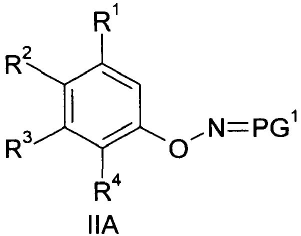
wherein:
PG¹ represents an imino-protecting group;
and R¹, R², R³ and R⁴ are as defined above,
characterised in that the reaction is performed in the presence of a hydrogen halide, phosphoric acid or sulfuric acid and a solvent system comprising at least 15% by weight of water,
which process is hereinafter referred to as "the process for the preparation of a compound of formula II".

Unless otherwise specified, the process for the preparation of a compound of formula II may be performed employing salts, solvates or protected derivatives, thereby producing compounds that may or may not be produced in the form of a (e.g. corresponding) salt or solvate, or a protected derivative thereof. However, the compound of formula II that is produced by the process for the synthesis of a compound of formula II necessarily contains an unprotected -ONH₂ group, given that the process involves a deprotection.

Compounds employed in or produced by the processes described herein (i.e. those involving the process of the invention or the process for the preparation of a compound of formula II) may exhibit tautomerism. The process of the invention and the process for the preparation of a compound of formula II therefore encompass the use or production of such compounds in any of their tautomeric forms, or in mixtures of any such forms.

Similarly, the compounds employed in or produced by the processes described herein (i.e. those involved in the process of the invention or the process for the preparation of a compound of formula II) may also contain one or more asymmetric carbon atoms and may therefore exist as enantiomers or diastereoisomers, and may exhibit optical activity. The process of the invention and the process for the preparation of a compound of formula II thus encompass the use or production of such compounds in any of their optical or diastereoisomeric forms, or in mixtures of any such forms.

Further, the compounds employed in or produced by the processes described herein (e.g. compounds of formula IIA as hereinbefore defined, which may exist as cis and trans isomers about the imino double bond) may contain double bonds and may thus exist as *E* (*entgegen*) and *Z* (*zusammen*) geometric isomers about each individual double bond.

Unless otherwise specified, alkyl groups as defined herein may be straight-chain or, when there is a sufficient number (i.e. a minimum of three) of carbon atoms be branched-chain, and/or cyclic. Further, when there is a sufficient number (i.e. a minimum of four) of carbon atoms, such alkyl groups may also be part cyclic/acyclic. Such alkyl groups may also be saturated or, when there is a sufficient number (i.e. a minimum of two) of carbon atoms, be unsaturated.

The term "aryl", when used herein, includes C₆₋₁₀ groups. Such groups may be monocyclic, bicyclic or tricyclic and, when polycyclic, be either wholly or partly aromatic. C₆₋₁₀ aryl groups that may be mentioned include phenyl and naphthyl.

For the avoidance of doubt, the point of attachment of substituents on aryl groups may be *via* any carbon atom of the ring system.

The term "heteroaryl", when used herein, includes 5- to 14-membered heteroaryl groups containing one or more heteroatoms selected from oxygen, nitrogen and/or sulfur. Such heteroaryl group may comprise one, two or three rings, of which at least one is aromatic. Substituents on heteroaryl groups may, where appropriate, be located on any atom in the ring system including a heteroatom. The point of attachment of heteroaryl groups may be *via* any atom in the ring system including (where appropriate) a heteroatom. Examples of heteroaryl groups that may be mentioned include pyridyl, pyrrolyl, quinolinyl, furanyl, thienyl, oxadiazolyl, thiadiazolyl, thiazolyl, oxazolyl, pyrazolyl, triazolyl, tetrazolyl, isoxazolyl, isothiazolyl, imidazolyl, pyrimidinyl, indolyl, pyrazinyl, indazolyl, pyrimidinyl, quinolinyl, benzoimidazolyl and benzthiazolyl.

The term "halo", when used herein, includes fluoro, chloro, bromo and iodo.

In the the process for the preparation of a compound of formula II, it is preferred that in compounds of formula IIA, PG¹ represents an imino-protecting group (i.e. a protecting group for the amino moiety that results in an imino functional group), such as =C(R^{q1})OR^{q2} (so forming a protected hydroxylamine group that is -O-N=C(R^{q1})OR^{q2}), in which R^{q1} and R^{q2} independently represent C₁₋₆ alkyl, and more preferably represent C₁₋₃ alkyl. Most preferably R^{q1} represents methyl and/or R^{q2} represents ethyl (so forming, for example, a compound of formula IIA in which the protected hydroxylamine group is -O-N=C(CH₃)OCH₂CH₃).

When used herein (e.g. in the context of protecting groups), the term "optionally substituted aryl" preferably refers to "optionally substituted phenyl", in which the optional substituents are preferably selected from halo, -NO₂, -OH and/or -OC₁₋₆ alkyl.

Preferably, in the process for the preparation of a compound of formula II, a compound of formula IIA in which PG¹ is as described herein and is most preferably =C(CH₃)(OCH₂CH₃), is deprotected to form a compound of formula II.

Preferred compounds of formula II that may be prepared include those in which: R¹, R², R³ and R⁴ independently represent hydrogen, halo, -NO₂, -CN, -C(O)₂R^{x1}, -N(R^{x6})R^{x7} or -N(R^{x10})S(O)₂R^{x11};
R^{x1}, R^{x2}, R^{x3}, R^{x6}, R^{x7}, R^{x8}, R^{x9} and R^{x10} independently represent hydrogen or C₁₋₄ alkyl optionally substituted by one or more halo atoms;
R^{x4}, R^{x5}, R^{x11} and R^{x12} independently represent C₁₋₄ alkyl optionally substituted by one or more halo (e.g. fluoro) atoms.

Further preferred compounds of formula II that may be prepared include those in which:
any three of R¹, R², R³ and R⁴ (preferably R¹, R³ and R⁴) represent hydrogen;
any one of R¹, R², R³ and R⁴ (preferably R²) represents a substituent selected from halo, -CN, -C(O)₂R^{x1}, preferably, -N(R^{X10})S(O)₂R^{x11} or, more preferably, -NO₂ or -N(R^{x6})R^{x7};
R^{x1} represents H or C₁₋₃ alkyl (e.g. propyl, such as isopropyl);
R^{x6}, R^{x7} and R^{x10} independently represent hydrogen;
R^{x11} represents C₁₋₂ alkyl (e.g. methyl).

Further preferred compounds of formula II that may be prepared include those in which R¹, R², R³ and R⁴ independently represent hydrogen or -NO₂. For example, any three of R¹, R², R³ and R⁴ (preferably R¹, R³ and R⁴) represent hydrogen and/or any one of R¹, R², R³ and R⁴ (preferably R²) represents -NO₂. Most preferably, R¹, R³ and R⁴ independently represent hydrogen; and/or R² represents -NO₂.

As stated above, the acid employed in the process for the preparation of a compound of formula II may be a hydrogen halide, phosphoric acid or sulfuric acid. The most preferred embodiment is one in which the process is performed in the presence of a hydrogen halide (e.g. HCl) and a solvent system (such as one described herein).

As described in more detail hereinafter, it is preferred that in the process for the preparation of a compound of formula II, the compound of formula IIA is added to the mixture of hydrogen halide, phosphoric acid or sulfuric acid (preferably hydrogen halide, e.g. HCl) and the solvent system employed. However, in such an instance the whole of the solvent system employed in the process of the reaction need not be mixed with the acid. For example, some of the solvent system may be mixed with the compound of formula IIA (which may aid its addition to the reaction, for example). Further, when organic solvent is present in the reaction mixture, then such solvent may be mixed with the acid, but is preferably mixed with the compound of formula IIA (in order to aid dissolution). However, at least 20% (e.g. at least 30%) of the water present in the solvent system is preferably first mixed with the acid that is employed (e.g. the hydrogen halide; which may exist as hydrogen halide in water as described hereinafter). Preferably, at least 50% (e.g. at least 60%, such as at least 75%) of water that is present in the solvent system is first in admixture with the acid (to which the compound of formula IIA, which may itself be present in solvent, is added).

Unexpectedly, the process for the preparation of a compound of formula II proceeds in the presence of a solvent system in which there is a reduced amount of organic solvent present (up to a negligible amount of organic solvent) as described herein, whereas it would be expected that the process requires the presence of an organic solvent in order to aid the dissolution of the compound of formula IIA that is to be deprotected. Surprisingly, however, the reaction proceeds in the presence of a reduced amount of organic solvent.

Advantageously, the order of addition of the reactants (i.e. the addition of the compound of formula IIA to the mixture of solvent system and acid) mentioned above has the additional advantage that the mixture of reactants involved in the process of reaction is one that is more easily handled, for example, the mixture may be a solution (or at least substantially in solution) or a substantially homogenous mixture that can be easily agitated. This is clearly advantageous from a practical point of view, as the reaction is allowed to proceed more easily (as there may be more interaction between the molecules of reactants, as opposed to when the reaction mixture is e.g. thicker or a slurry). Hence, this order of addition may allow the reaction to proceed in a higher yield.

The total amount of solvent employed in the process for the preparation of a compound of formula II should be sufficient for the reaction to proceed (e.g. at a predetermined rate, in order to maximise yield or minimise reaction time).

Hence, any suitable amount of solvent may be employed. Preferably, however, the amount of solvent employed in the process of the invention is at least 1%, e.g. at least 10% by weight of the compound of formula IIA (e.g. at least 25%, preferably, at least 50% by weight and especially at least 100% by weight) and/or at least 5% by weight of the acid (e.g. at least 25%, preferably, at least 50% by weight and especially at least 100% by weight) employed. Alternatively (and particularly when the solvent system comprises predominantly water, e.g. exclusively water), the total amount of solvent present is in an amount that is at least one molar equivalent, compared to the compound of formula IIA. Preferably, there is at least three molar equivalents of solvent present in the solvent system of the process of the invention, e.g. at least five molar equivalents. The actual amount/volume of solvent employed may be varied, depending on requirements of rate of reaction or yield.

There may be any upper limit of the amount of solvent required in the process. However, this may be determined practically so that the reaction mixture is not too dilute (e.g. such that the rate of reaction is too slow) or the quantity is so much that there is excess wastage.

As stated above, the process for the preparation of a compound of formula II is performed in the presence of a solvent system comprising at least 15% water (by weight). Preferably, the solvent system comprises at least 25% by weight of water, for example at least 50% by weight of water. More preferably, the solvent system comprises at least 70% (e.g. at least 80%) and, most preferably, at least 90% by weight water. Most preferably, the solvent system comprises at least 95% water (by weight) and consists of water (for instance, the solvent system consists predominantly of water (preferably, it consists exclusively of water), e.g. at or near 100% by weight of the solvent system comprises water). Hence, most preferably, the solvent system consists of water.

Provided that it comprises at least 15% water (by weight), the solvent system may also comprise an organic solvent, for example a polar solvent, such as a polar protic solvent, for example an alcohol (e.g. a C₁₋₆ alcohol, such as ethanol or, preferably, methanol), or, more preferably, a polar aprotic solvent such as dioxane, tetrahydrofuran, diethyl ether, dimethoxyethane or, most preferably, acetonitrile. Mixtures of the aforementioned solvents may also be employed.

In the process for the preparation of a compound of formula II, the solvent system comprises less than 85% by weight of an organic solvent, and preferably, less than 50% by weight of an organic solvent. More preferably, the process is performed in the presence of less than 30% (e.g. less than 20%, such as less than 10%) by weight of an organic solvent. Most preferably, less than 5% by weight of an organic solvent may be employed in the process for the preparation of a compound of formula II, for example, the processis performed in the absence of an organic solvent (i.e. in an amount by weight of less than 1% of an organic solvent, i.e. an insignificant amount).

The process for the preparation of a compound of formula II is particularly advantageous, as it may reduce (or eliminate) the use of an organic solvent in the process. This has several advantages including the associated environmental benefits, as well as practical benefits, such as the ease of separation of the product and the reduction of (or complete circumvention of) the removal of organic solvent employed in the process. Further, the reduction (or elimination) of organic solvent may also be of benefit economically (given that, for example, acetonitrile may be expensive).

Environment benefits include the reduction of any toxic by-products (e.g. nitrophenol) that may be formed as a consequence of employing an organic solvent. By reducing (or eliminating) the use of an organic solvent, surprisingly, the process for the preparation of a compound of formula II still proceeds efficiently, which is coupled with the advantages associated with the reduction (or elimination) of the organic solvent. Further, the process for the preparation of a compound of formula II may be accompanied by a corresponding reduction in the quantity of by-products (particularly toxic by-products), which may be linked to the corresponding reduction of the amount of organic solvent employed in the prowess.

In another aspect, the process for the preparation of a compound of formula II is performed as described herein, but in which the solvent system is one in which water is present in a molar ratio (compared to other solvents in the solvent system) of greater than 1:3, for example, the molar ratio of water:other solvent (in which the other solvent may be an organic solvent, such as an alcohol or, preferably, acetonitrile) is at least 1:2, for example at least 1:1, preferably 2:1. More preferably, the molar ratio of water:other solvent is at least 5:1, e.g. at least 10:1, and most preferably, the molar ratio is greater than 50:1 (for example, the solvent system comprises predominantly, or exclusively, water, as defined herein).

The hydrogen halide employed in the process of the invention may be HBr, HI, but is preferably HCl.

Preferably, in the process for the preparation of a compound of formula II, the acid (e.g. hydrogen halide), which may be in the presence of solvent (e.g. water) is mixed/reacted with the compound of formula IIA (which may, optionally be a mixture of compound of formula IIA and the solvent system, as defined herein, e.g. water). As stated herein, it is preferred that the compound of formula IIA is added to the acid (e.g. hydrogen halide), optionally in the presence of solvent (e.g. water). Preferably, at least one molar equivalent of hydrogen halide (e.g. HCl) is employed, for example, at least, 2 equivalents (preferably at least 3 equivalents, e.g. at least 4 equivalents such as 5 equivalents).

Preferably, the acid (e.g. hydrogen halide, such as HCl) employed in the process for the preparation of a compound of formula II is employed (e.g. as a hydrogen halide) in a solvent. Preferably, therefore, the acid (e.g. the hydrogen halide) is employed as a reagent in aqueous solution. It may be employed at any suitable concentration by weight (provided that a sufficient molar quantity is employed). However, preferably, it is employed as a solution (e.g. an aqueous solution) containing at least 10% (e.g. at least 20%, e.g. at least 30%, such as 37%) of acid (e.g. hydrogen halide) by weight. Advantageously, preferred concentrations of acid (e.g. hydrogen halide) may lead to *inter alia* the process of the reaction having a better rate of reaction, being more efficient and/or leading to a higher yield.

It is stated herein that the acid, e.g. hydrogen halide (which may be employed as hydrogen halide in an aqueous solution), is reacted/mixed with the compound of formula IIA. As stated herein, preferably, the compound of formula IIA is added to the acid (e.g. hydrogen halide), both of which may be present in solvent as described herein (e.g. the hydrogen halide is preferably present in an aqueous solution). This addition is preferably performed in portions over a period of time. For example, the compound of formula IIA may be added at such a rate as to maintain the temperature of the reaction (the process of the invention) at a certain level, for example near to room temperature (e.g. or as near as possible to room temperature). Preferably, the temperature is maintained below 50°C (e.g. between room temperature and 50°C), such as below 40°C, e.g. below 35°C. Most preferably, the temperature is maintained at between room temperature (25°C) and 32°C. The process for the preparation of a compound of formula II may also be performed at below room temperature, but is preferably performed above 0°C, and is most conveniently performed at room temperature.

The compound of formula IIA may be added to the acid (e.g. hydrogen halide) as a mixture in the solvent system employed in the process for the preparation of a compound of formula II. For example, it may be employed as a mixture of compound of formula IIA in water (for example, as described hereinbefore). The portion-wise addition of the compound of formula IIA to the acid, e.g. hydrogen halide, (or aqueous solution thereof) in the process for the preparation of a compound of formula II is most preferably effected by adding 1 mole of compound of formula IIA over a period of 1 hour (e.g. 0.8 moles over a period of 50 minutes). However, the addition need not be portion-wise, i.e. the addition can be substantially as a single "lump-sum". When the addition is portion-wise, then 1 mole of compound of formula IIA may be added to the acid (e.g. hydrogen halide) over a period of time of between ten minutes and two hours (and is most preferably over a preferred period of 1 hour, as indicated above). The portion-wise addition may be effected by a continuous addition process over the period of time required, for example, the addition may be *via* the continuous addition of a compound of formula IIA (in e.g. aqueous solvent) by means of a syringe pump, which may be set to perform the addition at the relevant rate required. The portion-wise addition may also be effected at pre-determined intervals (i.e. noncontinuous addition).

If the number of moles of compound of formula IIA in the process for the preparation of a compound of formula II is increased or decreased, then the period of time over which the addition occurs may be increased or decreased accordingly (for example, if two moles are employed, then the addition time may be doubled). However, the skilled person will appreciate that other factors may influence the necessary addition period (for example, concentration of the reagents in the solvent and/or temperature; higher concentrations and lower temperatures may reduce the addition period).

After the deprotection step of the process for the preparation of a compound of formula II has been effected, then the acidic medium of the reaction mixture may need to be neutralised. As the process for the preparation of a compound of formula II is performed in the presence of acid (e.g. a hydrogen halide, preferably, HCl), then the product of formula II so formed may exist as an acid (e.g. a hydrogen halide) salt of the compound of formula II.

In the context of this invention an acid (e.g. a hydrogen halide) salt of a compound of formula II refers to a compound formed by an association between a compound of formula II and the acid, such as hydrogen halide (e.g. HCl). The association between these two moiteies may be any kind of physico-chemical association (i.e. interaction or bonding) between the respective moieties, for example an ionic association (wholly or in part), so forming a salt, or one or more other kinds of association (wholly or in part), such as a covalent (including polar covalent and coordinate covalent) association, a metallic association, or another, electrostatic association, such as a permanent dipole to permanent dipole interaction, hydrogen bonding, van der Waals forces and/or a cation-pi interaction. However, preferably, the association is at least partly ionic, so forming a salt.

Any acid (e.g. hydrogen halide) salt of the compound of formula II formed by the process for the preparation of a compound of formula II may be neutralised under standard conditions. For example in the presence of a suitable base, for an alkali metal based base, such as an alkali metal hydroxide (preferably sodium hydroxide). For example, the base (e.g. aqueous sodium hydroxide solution), may be between 10 and 50% w/w, e.g. between 15 and 40% w/w, e.g. 33% w/w). Preferably, the base is added to the mixture of the products of the process of the invention at such a rate at to maintain the temperature of the mixture at a certain level (such as below 50°C), for example, it is maintained at the same level as the temperature is maintained during the process for the preparation of a compound of formula II, i.e. the temperature is most preferably maintained at between room temperature (25°C) and 32°C.

Such a neutralisation step, which is encompassed by the scope of the process for the preparation of a compound of formula II, advantageously produces the free-base of the compound of formula II, which may precipitate out of the solvent system (which may comprise the solvent system employed in the process of the invention, e.g. water, and/or any additional solvent employed in the neutralisation step described herein, e.g. water). Hence, the free-base of the compound of formula II so formed may be isolated by standard techniques, e.g. filtration.

The present invention relates to a process for the preparation of a compound of formula I. 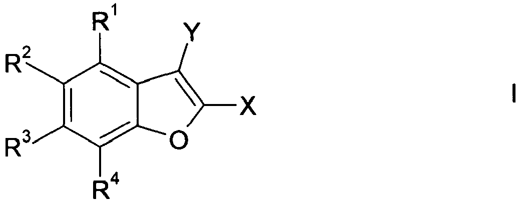 wherein R¹, R², R³ and R⁴ independently represent hydrogen, halo, -NO₂, -CN, -C(O)₂R^{x1}, -OR^{x2}, -SR^{x3}, -S(O)R^{x4}, -S(O)₂R^{x5}, -N(R^{x6})R^{x7}, -N(R^{x8})C(O)R^{x9}, -N(R^{x10})S(O)₂R^{x11} or R^{x12};
X represents hydrogen or C₁₋₆ alkyl optionally substituted by one or more halo atoms;
Y represents -C(O)-Z;
Z represents aryl or heteroaryl, both of which are optionally substituted by one or more substituents selected from -OR^{a}, halo, -NO₂, -CN, -C(O)₂R^{a1}, -SR^{a3}, -S(O)R^{a4}, -S(O)₂R^{a5} -N(R^{a6})R^{a7}, -N(R^{a8})C(O)R^{a9}, -N(R^{a10})S(O)₂R^{a11} and R^{a12};
R^{a} represents an oxy-protecting group, hydrogen or C₁₋₆ alkyl optionally substituted by one or more substituents selected from halo, -C(O)₂R^{b1} and -N(R^{b2})R^{b3};
R^{x1}, R^{x2}, R^{x3}, R^{x6}, R^{x7}, R^{x8}, R^{x9}, R^{x10}, R^{a1}, R^{a3}, R^{a6}, R^{a7}, R^{a8}, R^{a9}, R^{a10}, R^{b1}, R^{b2} and R^{b3} independently represent hydrogen or C₁₋₆ alkyl optionally substituted by one or more halo atoms;
R^{x4}, R^{x5}, R^{x11}, R^{x12}, R^{a4}, R^{a5}, R^{a11} and R^{a12} independently represent C₁₋₆ alkyl optionally substituted by one or more halo atoms;
wherein the process comprises reaction of a compound of formula II, as prepared by the process for the preparation of a compound of formula II hereinbefore defined, 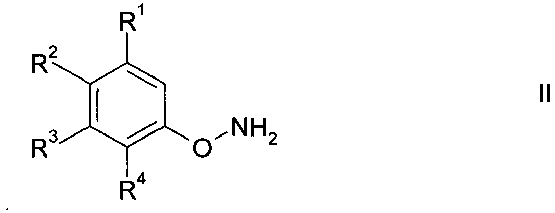 wherein:
R¹, R², R³ and R⁴ independently represent hydrogen, halo, -NO₂, -CN, -C(O)₂R^{x1} -OR^{x2}, -SR^{x3}, -S(O)R^{x4}, -S(O)₂R^{x5}, -N(R^{x6})R^{x7}, -N(R^{x8})C(O)R^{x9}, -N(R^{x10})S(O)₂R^{x11} or R^{x12};
R^{x1}, R^{x2}, R^{x3}, R^{x6}, R^{x7}, R^{x8}, R^{x9} and R^{x10} independently represent hydrogen or C₁₋₆ alkyl optionally substituted by one or more halo atoms;
R^{x4}, R^{x5}, R^{x11} and R^{x12} independently represent C₁₋₆ alkyl optionally substituted by one or more halo atoms;
with a compound of formula III, 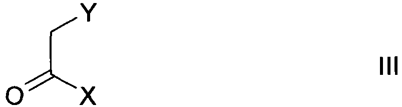 wherein Y and X are as defined above,
and wherein the process further comprises preparation of the compound of formula II *via* a process comprising deprotection of a compound of formula IIA, 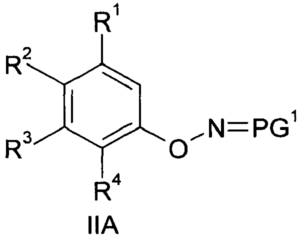 wherein:
PG¹ represents an imino-protecting group;
and R¹, R², R³ and R⁴ are as defined above,
characterised in that the deprotection of a compound of formula IIA is performed in the presence of a hydrogen halide, phosphoric acid or sulfuric acid and a solvent system comprising at least 25% by weight of water.

This process is hereinbefore and hereinafter referred to as "the process of the invention".

In a further embodiment of the invention, there is provided a process for the preparation of a compound of formula I as hereinbefore defined, but characterised in that:
Y represents -C(O)Z, which process comprises reaction of a compound of formula II prepared by the process of the invention as hereinbefore defined, with a compound of formula III as hereinbefore defined, but in which Y represents -C(O)Z;
the reaction is performed as a "one-pot" procedure;
R² represents -NO₂, which process comprises reaction of a compound of formula II prepared by the process as hereinbefore defined, but in which R² represents -NO₂, with a compound of formula III as hereinbefore defined; or
the process is performed in the absence of an acylating reagent (for example, when the process of the invention proceeds *via* an intermediate of formula XXIV (as defined hereinafter), then that intermediate is not first reacted in the presence of an acylating reagent (such as trifluoroacetic anhydride or trifluoroacetyl triflate) to form an *N*-acylated intermediate in order to promote the pericyclic cyclisation to form the compound of formula I).

The above-mentioned embodiments of the invention are also referred to herein as the "process of the invention".

Also described herein, is a process for the preparation of a compound of formula I as hereinbefore defined wherein Y = H.

It is stated herein that R^{a} may represent an oxy-protecting group. Oxy-protecting groups that may be mentioned include trialkylsilyl and diarylalkyl-silyl groups (e.g. *tert-*butyldimethylsilyl, *tert*-butyldiphenylsilyl or trimethylsilyl), tetrahydropyranyl, -C(O)R^{t1}, C₁₋₆ alkyl (which alkyl group is optionally substituted by one or more substituents selected from optionally substituted aryl, so forming an alkylaryl group), -S(O)₂R^{t2}, -C(O)OR^{t3} and -C(O)N(R^{t4})R^{t5}, in which R^{t1}, R^{t2}, R^{t3}, R^{t4} and R^{t5}, as well as preferred optional substituents on any relevant aryl groups, are as hereinbefore defined. The skilled person will appreciate that in compounds of formula I, when R^{a} represents C₁₋₆ alkyl, certain of these groups may be considered to be protecting groups (e.g. allylic groups). Other oxy-protecting groups include salts, for example an inorganic metal salt, such as a group II or, preferably a group I metal salt (e.g. a sodium or potassium salt, so forming for example a -O⁻Na⁺ or -O⁻K⁺ moiety).

Most preferred oxy-protecting groups include -C(O)R^{t1} groups, preferably in which R^{t1} represents a C₁₋₆ alkyl group, so forming an alkylcarbonyl groups (e.g. methyl-and ethylcarbonyl groups), and alkylaryl groups (e.g. benzyl optionally substituted as hereinbefore defined). It is most preferred that, when R^{a} represents an oxy-protecting group, then it represents an alkylaryl group, especially a benzyl group, which is optionally substituted as defined herein, but preferably unsubstituted.

Compounds of formula IIA (employed in the process of the invention) may be prepared by reaction of a compound of formula IV, 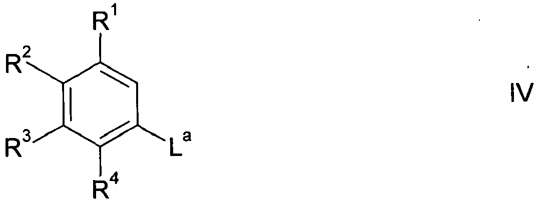 wherein L^{a} represents a suitable leaving group, such as a sulfonate group (e.g. -OS(O)₂CF₃, -OS(O)₂CH₃ or -OS(O)₂PhMe) or, more preferably halo (e.g. bromo, fluoro or, preferably, chloro), and R¹, R², R³ and R⁴ are as hereinbefore defined, with a compound of formula V,

HO-N=PG¹ V

wherein PG¹ is as hereinbefore defined, for example under standard aromatic substitution reaction conditions. For instance, the aromatic substitution reaction may be performed in the presence of a polar aprotic solvent (such as dimethylformamide). In this context, other polar aprotic solvents that may be mentioned include tetrahydrofuran, dimethylsulfoxide, diethyl ether and dioxane. However, it has now been found that this process step may also be performed in a mixture of solvents, only one of which is a polar aprotic solvent (and the other is a non-polar solvent). Hence, in another aspect of the invention, there is provided such a process in the presence of a non-polar solvent, such as a non-polar aprotic solvent, which solvent is employed in addition to the polar aprotic solvent as defined above (and which is preferably dimethylformamide). Preferred non-polar aprotic solvents include toluene, but may be any solvent that may be employed to extract compounds of formula V (e.g. from a reaction mixture as defined hereinafter).

It is preferred that the compound of formula V is protected. This is because otherwise, this may lead to non-regioselective nucleophilic aromatic substitution onto the aromatic ring of the compound of formula IV, i.e. a compound in which the nitrogen atom of hydroxylamine is linked to the aromatic ring (rather than the oxygen atom).

Advantageously, in this instance (i.e. the process for the preparation of compounds of formula IIA), a solution containing the compound of formula V (whichever is employed), for example a solution obtained by the extraction from a reaction mixture (following the preparation of those compounds of formula V), need not be concentrated by the partial or complete evaporation of the solvent (i.e. advantageously, solvent need not be removed). Rather, a polar aprotic solvent (e.g. DMF) may preferably be added directly to a solution of the compound of formula V without complete removal (and most preferably, without any removal) of any non-polar solvent, for example that which is employed in an extraction.

Compounds of formula III in which Y represents -C(O)-Z may be prepared by:
(i) reaction of a compound of formula VII,

   Z-C(O)-CH₃ VII

   wherein Z is as hereinbefore defined, with a compound of formula VIII,

   X-C(O)-L¹ VIII

   wherein L¹ represents a suitable leaving group, such as halo (e.g. bromo, chloro or iodo) or, more preferably, -OC₁₋₆ alkyl (e.g. -OCH₃ or, preferably, -OCH₂CH₃), and X is as hereinbefore defined, preferably in the presence of a suitable base, such as an alkali metal hydride (e.g. KH, CaH₂ or, preferably, NaH), an organolithium base (e.g. *n*-, *s*- or *t*-butyllithium or, preferably, lithium diisopropylamide), another alkali metal based base (e.g. Na₂CO₃, K₂CO₃, K₃PO₄, *t*-BuONa, *t*-BuOK or, preferably, CH₃ONa), or mixtures of bases, and (a) suitable solvent(s) (such as tetrahydrofuran (THF), toluene and/or dimethylformamide; a polar aprotic solvent such as THF is particularly preferred) under standard conditions, such as at room temperature or elevated temperature, such as 65°C;
(ii) reaction of a compound of formula IX,

   X-C(O)-CH₃ IX

   wherein X is as hereinbefore defined, with a compound of formula X,

   Z-C(O)-L¹ X

   wherein Z and L¹ are as hereinbefore defined, for example under reaction conditions such as those hereinbefore described in respect of preparation of compounds of formula III (process step (i) above);
(iii) for compounds of formula III, in which Y represents -C(O)-Z and Z represents aryl or heteroaryl substituted by -OH, reaction of a corresponding compound of formula XI,

   H₃C-C(O)-Z^{a} XI

   wherein Z^{a} represents aryl or heteroaryl substituted with -O-C(O)-X (in which X is as hereinbefore defined), with base, for instance a base and reaction conditions such as those hereinbefore defined in respect of preparation of compounds of formula III (process step (i) above). For the avoidance of doubt, the -O-C(O)-X substituent of the compound of formula XI is converted to the -OH substituent of the compound of formula III;
(iv) decarboxylation of a compound of formula XII, 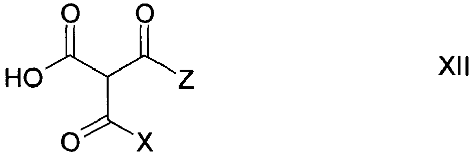 or a protected (e.g. a -C(O)OH protected) derivative thereof (such as an ester of a -C(O)OH), wherein X and Z are as hereinbefore defined, under standard decarboxylation reaction conditions known to those skilled in the art;
(v) hydrolysis of a compound of formula XIII, 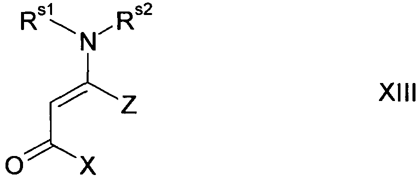 wherein R^{s1} and R^{s2} independently represent hydrogen, C₁₋₆ alkyl optionally substituted by one or more halo atoms, or R^{s1} and R^{s2} are linked together to form, together with the nitrogen atom to which they are necessarily attached, a 4- to 8-membered (e.g. 5- or 6-membered) heterocycloalkyl group (optionally containing a further heteroatom, such as a further nitrogen or oxygen heteroatom, and which heterocycloalkyl group is optionally substituted by one or more substituents selected from halo or C₁₋₆ alkyl), such as a piperidinyl or pyrrolidinyl ring, and X and Z are as hereinbefore defined, under standard conditions, for example in the presence of an aqueous acid (e.g. an aqueous solution of a hydrogen halide);
(vi) for compounds of formula III in which Z preferably represents aryl (e.g. phenyl) substituted (preferably in the *ortho-* or, more preferably in the *para* position) with -SR^{a3}, -N(R^{as})R^{a7} or, preferably, -OR^{a}, reaction of a compound of formula XIV,

   Z-H XIV

   wherein Z is as hereinbefore defined, and preferably represents aryl (e.g. phenyl) substituted (preferably in the *ortho-* or, more preferably in the *para* position) with -SR^{a3}, -N(R^{a6})R^{a7} or, preferably, -OR^{a} and R^{a}, R^{a3}, R^{as} and R^{a7} are as hereinbefore defined, with either:
   (A) a compound of formula XV,

      X-C(O)-CH₂-C(O)-L¹ XV

      or a protected derivative (e.g. acetal) thereof, wherein X is as hereinbefore defined, and L¹ is as hereinbefore defined and preferably represents halo (e.g. bromo or, preferably, chloro); or
   (B) a compound of formula XVI,

      X-C(O)-CH₂-CN XVI

      or a protected derivative (e.g. acetal) thereof, wherein X is as hereinbefore defined,
   under standard reaction conditions known to those skilled in the art, for instance under Friedel-Crafts acylation reaction conditions, e.g. in the presence of a suitable acid such as a protic acid (e.g. sulfuric acid) or, preferably, a Lewis acid such as AlCl₃. The skilled person will appreciate that when a protected derivative (e.g. an acetal protected derivative) of a compound of formula XV or XVI is employed, the resultant compound of formula III may need to be deprotected under standard conditions. Protecting groups that may be employed include acetals, which may protect any carbonyl group present. Acetal derivatives of compounds of formula XV or XVI that may be mentioned include compounds of formula X-C(OR^{v1})₂-CH₂-C(O)-L¹ and X-C(OR^{v1})₂-CH₂-CN, in which each R^{v1} independently represents C₁₋₆ alkyl, or, the two R^{v1} groups may be linked together to form, together with the oxygen atoms to which they are necessarily attached, a 4- to 7-membered (e.g. 5- or 6-membered) ring (i.e. a cyclic acetal). Such acetal protecting groups may be introduced by the reaction of a compound of formula XV or XVI in the presence of an appropriate alcohol (e.g. of formula HO-R^{v1}) or a diol (e.g. of formula HO-R^{v1}-R^{v1}-OH, in which the relevant R^{v1} groups are linked together) in the case of the formation of cyclic acetals, under appropriate acid or base catalysis conditions. Such acetal protecting groups may be removed under standard conditions, for example by hydrolysis e.g. in the presence of acid;
(vii) reduction of a compound of formula XVIA, 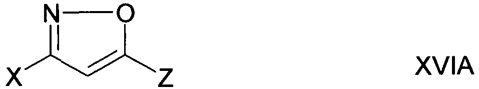 or a compound of formula XVIB, 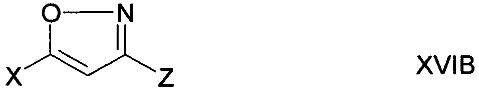 wherein (in both cases) X and Z are as hereinbefore defined, in the presence of aqueous acid, under standard conditions, for example reduction by hydrogenolysis, which may be performed in the presence of a suitable catalyst system. The catalyst may be a precious transition metal, for example platinum, ruthenium, nickel (e.g. Raney nickel) or, especially, palladium. The metal may be used as such in powder form, as its oxide or hydroxide or, preferably, on a suitable support, such as powdered charcoal. Typically, palladium on charcoal is used (e.g. 5% Pd/C). Advantageously, when there is another group present that requires reduction to form the compound of formula III, then two steps may be performed in "one-pot". For instance, when Z represents aryl or heteroaryl substituted by -OR^{a} in which R^{a} represents a protecting group susceptible to cleavage *via* a hydrogenolysis reaction, e.g. a benzyl protecting group, then such a group may also be cleaved by such a hydrogenolysis reaction to form a corresponding -OH group, at the same time as the isoxazole moiety undergoes hydrogenolysis to the appropriate diketone (of formula III).

Advantageously, compounds of formula III in which Y represents -C(O)Z (and Z represents aryl or heteroaryl substituted by at least one (e.g. one) -OH group) and X represents hydrogen or C₁₋₆ alkyl optionally substituted by one or more halo (e.g. fluoro) atoms may be prepared by reaction of a compound of formula VIIA,

Z-C(O)-CH₃ VIIA

wherein Z represents aryl or heteroaryl substituted by at least one (e.g. one) -OH group, characterised in that the requisite -OH substituent thereon is not protected, with a compound of formula VIII,

X-B¹ VIIIA

wherein:
X is as defined above;
B¹ represents -C≡N or, preferably, -C(O)L¹;
L¹ is a suitable leaving group, such as halo (e.g. bromo, chloro or iodo) or, more preferably, -OC₁₋₆ alkyl (e.g. -OCH₃ or, preferably, -OCH₂CH₃), in the presence of base, wherein the base comprises an alkali metal alkoxide, in which the alkyl moiety of the alkoxide is a branched C₃₋₆ alkyl group (i.e. equivalents of such a base), which is also referred to hereinafter as a process of the invention.

Such a reaction is characterised in that in the compound of formula VIIA, the requisite -OH substituent on the aryl or heteroaryl group defined by the integer Z is not protected. By this we mean that that group exists as a free -OH group or, in another embodiment, as a salt thereof, such as a moiety of formula -O⁻A⁺ in which A represents a Group I alkali metal, e.g. potassium or, preferably sodium, so forming e.g. a -O⁻Na⁺ moiety (however, the -OH group is not covalently bonded to another atom, such as a carbon atom). Preferably therefore, in the compound of formula III that is produced, the corresponding -OH is also not protected (but may exist as -O⁻A⁺ or in the free -OH form; in practice, the reaction will be quenched with a proton and hence any compound of formula III formed *in situ* in which there is a -O⁻A⁺ present may be converted to, and isolated as, a corresponding compound of formula III in which there is a free -OH group present). Such a process may be performed employing salts, solvates or protected derivatives (e.g. in which the carbonyl group is protected, as an imine) of the compounds of formulae VIIA and VIIIA. Compounds of formula III that may thereby be produced may or may not be produced in the form of a (e.g. corresponding) salt or solvate, or a protected derivative thereof (for example a protected carbonyl group, such as an imine may be produced). However, as stated hereinbefore, the requisite -OH substituent attached to the aryl or heteroaryl group in the Z group of the compound of formula VIIA may not be 'derivatised', i.e. it may not be protected (e.g. by being covalently bonded *via* a carbon atom), but exists as the free -OH group (or salt thereof). The skilled person will appreciate that when a compound of formula VIIIA in which B¹ represents -C≡N is employed, then the resultant product of formula III so formed may necessarily be one in which a carbonyl group is protected as an imine (e.g. a compound of formula III that is X-C(=NH)-CH₂-C(=O)-Z, or a derivative, or the like may be formed), in which the imino (=NH) moiety may be hydrolysed to give a compound of formula III that is X-C(=O)-CH₂-C(=O)-Z. Most preferably, a compound of formula VIIIA in which B¹ represents -C(O)L¹ is employed in the process of the invention.

In the process to prepare compounds of formula III, preferred compounds of formula III that may be produced include those in which:
X represents C₁₋₄ alkyl (optionally substituted by one or more fluoro atoms; but preferably, unsubstituted), for example C₄ alkyl, such 1-methylpropyl, or, most preferably, butyl (especially *n*-butyl);
Z represents phenyl substituted by one -OH group (or a salt thereof, e.g. a -O⁻Na⁺ group) in the 2-, 3- or, preferably, in the 4-position;
L¹ preferably represents a suitable leaving group such as halo (e.g. bromo, chloro or iodo) or, more preferably, -OC₁₋₆ alkyl (e.g. -OCH₃ or, preferably, -OCH₂CH₃); however, equivalent leaving groups may be employed.

In the process to prepare compounds of formula III by reaction of a compound of formula VIIA and VIIIA, the reaction is performed in the presence of a certain alkali metal alkoxide. Preferably, the alkali metal is a Group I metal, such as potassium or, preferably sodium. It is stated that the alkoxy moiety of the base is branched. Preferably, the branching occurs at the position a to the carbon atom that is attached to the requisite oxygen atom of the alkoxy group (and hence, the C₃₋₆ alkyl group is secondary or, preferably, tertiary, relative to the point of attachment to the oxygen atom). Most preferably, the alkoxy moiety is branched C₄₋₆ alkyl (e.g. *tert*-butyl). The most preferred base is sodium *tert*-butoxide. Such bases in which alkyl moiety of the alkali metal alkoxide is branched possess a higher pKa (i.e. are stronger bases) than corresponding bases in which the alkyl moiety is not branched, but linear (corresponding bases containing a primary alkyl group, relative to the point of attachment to the oxygen atom).

The base employed in the process to prepare compounds of formula III is one that possesses a certain pKa. Similarly, other suitable bases that possess a similar, or higher, pKa may also be employed in the process of the invention (which bases are referred to herein as equivalent bases to the requisite alkali metal alkoxide base employed in). Such bases are advantageous, as they may improve the yield and efficiency of the process, for example by reducing side reactions and therefore undesired by-products (e.g. reducing competing condensation reactions, e.g. self-condensations). When the compound of formula VIIA contains a free -OH group, this (i.e. the reduction of side reactions) may be due to accompanying deprotonation of that hydroxy group, which forms an alkali metal salt (i.e. -O⁻A⁺), which may make it less reactive to carbonyl groups, thereby decreasing the likelihood of self-condensation.

As stated hereinbefore, a certain alkali metal alkoxide is employed to prepare compounds of formula III or another suitable base (e.g. equivalent base).

Other suitable bases that may be employed include any of the following: another alkali metal based base (e.g. a carbonate base, such as Na₂CO₃ or K₂CO₃ and/or a phosphate base, such as K₃PO₄), an alkali metal hydride (e.g. KH, CaH₂ or, preferably, NaH), an organolithium base (e.g. *n*-, *s*- or *t*-butyllithium or, preferably, lithium diisopropylamide), or mixtures of bases.

For example when the compound of formula VIIA contains a free -OH group, it is preferred that at least, or, one equivalent of base (e.g. the requisite alkali metal alkoxide) is employed (equivalent to the molar quantity of the compound of formula VIIA). However, as the first equivalent of base may deprotonate the free -OH group of the compound of formula VIIA (thereby forming a corresponding compound of formula VIIA in which there is a -O⁻A⁺ moiety present), then it is preferred that at least 1.5 and preferably at least, or, 2 equivalents of base are employed, if yield is to be maximised. Most preferably, however, at least 2.5, e.g. at least, or, 3 equivalents of base (e.g. the requisite alkali metal alkoxide) is employed, in order to maximise yield, as the compound of formula III to be formed may enolise, and therefore may require an additional one equivalent of base. Preferably, all of the base employed in the process of the reaction is the requisite alkali metal alkoxide, or equivalent thereof, as defined herein. However, mixtures of different bases may be employed, provided that at least one equivalent, e.g. at least 2 (and preferably at least 3) equivalents of the requisite alkali metal alkoxide is employed.

When the compound of formula VIIA contains a -O⁻A⁺ moiety (instead of the free -OH group, in which A⁺ is a group I metal anion, preferably, Na⁺) then one less equivalent of base may be required (as the free -OH moiety has already been deprotonated), and hence, the amount of base (e.g. the requisite alkali metal alkoxide) is preferably, at least, or, one equivalent, and preferably, at least 2 equivalent. As stated hereinbefore, the compound of formula VIIA in which there is a -O⁻A⁺ moiety present may be prepared in *situ* by reaction with the requisite alkali metal alkoxide base present in the process of the reaction. However, such a compound may be pre-formed, or may be formed *in situ* by reaction with another suitable alkali metal base first (followed by the reaction with the compound of formula VIIIA and requisite alkali metal alkoxide base, or equivalent), in which case suitable bases include alkali metals (such as sodium, e.g. sodium wire) or strong alkali metal bases such as alkali metal hydroxides (e.g. potassium or, preferably, sodium hydroxide; in which latter case a -O-Na⁺ moiety is formed).

The process to prepare compounds of formula III may be performed in the presence of (a) suitable solvent(s) (such as tetrahydrofuran (THF), toluene and/or dimethylformamide; a polar aprotic solvent such as THF is particularly preferred). However, in the case where one of the reactants (e.g. compound of formula VIIIA) is a liquid at the reaction temperature, then the reaction may also be performed in the absence of solvent (as the reactant, e.g. compound of formula VIIIA, may serve as solvent). As stated hereinbefore, the product (of compound III) formed by the process to prepare compounds of formula (III) may be in the form of an enolate. Hence, the reaction of this process is preferably quenched by the addition of an appropriate quantity (e.g. at least one equivalent) of a proton source, e.g. a protic acid, such as a hydrogen halide (e.g. HCI) or a weak organic acid (e.g. a carboxylic acid, such as acetic acid). Advantageously, when a weak organic acid is employed, the quench may be also result in crystallisation/precipitation of the product, for example, as defined hereinafter.

The process to prepare compounds of formula III may be performed in the presence of any quantity of each of the compounds of formulae VIIA and VIIIA. However, it is preferably performed in the presence of compounds of formulae VIIA and VIIIA that are in a molar ratio of from 3:2 to 2:3, and most preferably in a molar ratio of from 1.1:1 to 1:1.1 (e.g. 1:1). The process (i.e. to prepare compounds of formula III) may be performed under standard reaction conditions, such as at room temperature or elevated temperature (e.g. 40°C), such as 65°C, or above (e.g. between 40°C and 85°C). Preferably, such a reaction is performed in the absence of a further additive such as a boron reagent (such as BF₃ or BF₂, or a complex thereof). Further, the compound of formula III produced is not isolated as a complex, for example a copper chelates.

Also described herein, there is provided a (not falling under the scope of the invention) process for the isolation/purification of a compound of formula III, as hereinbefore defined but in which Y represents - C(O)Z (and preferably, X and Z are as hereinbefore defined), which process comprises crystallisation or precipitation of the compound, in a solvent system. Crystallisation (or precipitation) of the compounds prepared may be performed in any suitable solvent (or mixtures of solvents). However, it has preferably surprisingly been found that certain solvent systems are particularly preferred. Particularly preferred solvent systems for the crystallisation or precipitation of the compound of formula III include an aqueous solvent and weak organic acids (such as a carboxylic acid as defined herein, e.g. formic, propionic, or preferably, acetic acid). The crystallisation/precipitation process described herein has the additional advantage that the compound of formula III may be present in the reaction mixture with other products (e.g. unreacted starting material or other undesired side-products), but this purification/isolation process may still proceed. For example, the compound of formula III may be present in less than 95% (e.g. less than 90%, such as less than, or, 80%) of the mixture to be crystallised/precipitated, but the isolated/purified product so formed may not contain those undesired products (and may be present in a higher percentage, such as above 95%, e.g. above 99%, such as near, or at, 100%, in the product formed). Most preferably, the solvent system employed in the crystallisation or precipitation process comprises a mixture of water and a weak organic acid (e.g. acetic acid). When such a mixture of solvents is employed in the solvent system, then any ratios may be employed, for instance, between 1:10 and 10:1 of water:weak organic acid. However, preferably, the ratio is between 1:5 and 5:1, for example between 1:3 and 3:1 and, especially, 1:1. Preferably, the crystallisation solvent is homogenous, for example the solvents may forms an azeotropic mixture. However, a suitable solvent may also be employed as an "anti-solvent" (i.e. a solvent in which salts of compounds of formula I are poorly soluble) in order to aid the crystallisation process. Crystallisation temperatures and crystallisation times depend upon the concentration of the compound in solution, and upon the solvent system which is used. Surprisingly, it has been found that the crystallisation or precipitation produces a new physical form of a compound of formula III. Hence, there is provided a compound of formula III obtainable by the crystallisation described herein. Also described herein, there is provided a compound of formula III (not falling under the scope of the invention) as hereinbefore defined
wherein the average particle size is at least 250 x 150 µM. Preferably, the average particle size is at least 300 x 200 µM (e.g. at least 400 x 300 µM, for example 500 x 380 µM). Such compounds may be inherently larger than those described in the prior art. "Average" when referred to herein refers to the median.

The new physical form (with increased average particle size) may lead to advantages in terms of handling of the compound of formula III and/or improvements in the characteristics of the compound.

Also described herein, there is provided a combination of the processes described herein. For example, there is provided a process for the preparation of a compound of formula III (which comprises reaction of a compound of formula VIIA and VIIIA, as hereinbefore defined; referred to hereinafter as process (i)) followed by crystallisation (or precipitation) as hereinbefore described (referred to hereinafter as process (ii)). Preferably, process (ii) is performed directly after process (i), for example, by separation of the compound of formula III (e.g. by extraction and removal/evaporation of solvent), following by mixing/contacting the compound of formula III with the solvent system of the crystallisation process. Alternatively, process (ii) can be performed directly after process (i) and in the same reaction pot, e.g. by quenching process (i) in the solvent system required for process (ii).

Compounds of formula V in which PG¹ represents =C(R^{q1})OR^{q2}, may be prepared by reaction of hydroxylamine, or a salt thereof (e.g. a hydrogen halide salt, such as HCl) with a compound of formula XVII,

HN=C(R^{q1})OR^{q2} XVII

wherein R^{q1} and R^{q2} are as hereinbefore defined, under standard reaction conditions. The reaction mixture to obtain such a product may be extracted with a suitable solvent, such as a non-polar solvent (e.g. toluene).

Compounds of formula XI may be prepared by reaction of a compound of formula XVIII,

H₃C-C(O)-Z^{b} XVIII

wherein Z^{b} represents aryl or heteroaryl substituted with -OH, with a compound of formula VIII as defined above, under standard conditions, for example, such as those described hereinbefore in respect of preparation of compounds of formula III (process step (i) above).

Compounds of formula XII may be prepared by reaction of a compound of formula X as defined above, with a compound of formula XIX,

X-C(O)-CH₂-C(O)OH XIX

or a protected (e.g. a -C(O)OH protected) derivative thereof (such as an ester of a -C(O)OH), wherein X is as hereinbefore defined, under standard reaction conditions, for example such as those hereinbefore described in respect of preparation of compounds of formula III (process step (i) above).

Compounds of formula XIII may be prepared by reaction of a compound of formula XX, 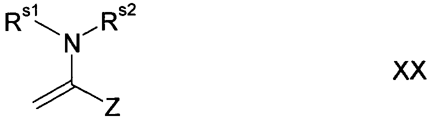 wherein Z, R^{s1} and R^{s2} are as hereinbefore defined, with a compound of formula VIII as hereinbefore defined, under reaction conditions such as those hereinbefore described in respect of preparation of compounds of formula III (process step (i)), and preferably in which, when a base is employed, it is a weak base, such as Na₂CO₃, K₂CO₃, K₃PO₄, *t*-BuONa, *t*-BuOK, preferably, CH₃ONa, or mixtures thereof.

Compounds of formula XVIA and XVIB may be prepared by reaction of corresponding compounds of formula III in which Y represents -C(O)-Z with hydroxylamine (or a salt thereof, e.g. HCl), under standard condensation reaction conditions. Such a process step starts with compounds of formula III, and hence when such a process step is taken in conjunction with process step (vii) above (in respect of preparation of compounds of formula III), then the resultant products are also compounds of formula III. Such a sequence of steps, however, are useful e.g. in obtaining compounds of formula III in a purer form. Therefore, these two steps taken in conjunction may provide a process for the purification (by which we mean the removal of any impurity, such as most of the impurities, including residual reactants) of compounds of formula III.

Compounds of formula XVII may be prepared by reaction of a compound of formula XXI,

R^{q1}-CN XXI

wherein R^{q1} is as hereinbefore defined, with a compound of formula XXII,

R^{q2}-OH XXI

wherein R^{q2} is as hereinbefore defined, under standard reaction conditions, for example, in the presence of an acid, such as a hydrogen halide (e.g. HCl).

Compounds of formula XX may be prepared by reaction of a compound of formula VII as defined above, with a compound of formula XXIII,

HN(R^{s1})R^{s2} XXIII

wherein R^{s1} and R^{s2} are as hereinbefore defined, under dehydration standard reaction conditions, e.g. in the presence of an appropriate acid catalyst (e.g. a non-aqueous acid, such as para-toluene sulfonic acid, or the like).

Compounds of formulae IV, VII, VIII, IX, X, XIV, XV, XVI, XVIII, XIX, XXI, XXII and XXIII (and certain other compounds, for instance, certain compounds of formulae II, III and V), may be commercially available, are known in the literature or may be obtained by conventional synthetic procedures, in accordance with known techniques, from readily available starting materials using appropriate reagents and reaction conditions.

Any of the processes described herein may advantageously be employed in conjunction (i.e. in sequence). For example, processes for the preparation of compounds of formula IIA may consist of, first, a process for the preparation of a compound of formula V as described herein (i.e. comprising reaction of a compound of formula XVII with hydroxylamine, or a salt thereof), followed by a process for the preparation of the compound of formula IIA (i.e. comprising reaction of a compound of formula IV with a compound of formula V so prepared), and then the compound of formula IIA may be employed to obtain the compound of formula II as hereinbefore defined (i.e. by deprotection in accordance with the procedures described herein).

Substituents on compounds of formula I, II, III, or any relevant intermediate compounds to such compounds (or solvates), for instance substituents defined by R¹, R², R³, R⁴, or substituents on Z, may be modified one or more times, before, after or during the processes described above by way of methods that are well known to those skilled in the art. Examples of such methods include substitutions, reductions, oxidations, alkylations, acylations, hydrolyses, esterifications, etherifications, halogenations, nitrations, diazotizations or combinations of such methods. In this manner certain compounds of formula I, II or III may be converted to other compounds of formula I, II or III respectively. For instance, a compound of formula IV in which R² represents -NO₂ may be employed (which compound may be better suited to a nucleophilic aromatic substitution reaction of a compound of formula IV with a compound of formula V) to synthesis a compound of formula IIA in which R² is also -NO₂. However, such a -NO₂ group may be reduced to an amino group before or after the process of the invention to form a corresponding compound of formula I in which R² represents amino. Such an amino group may not have been suited to the above-mentioned nucleophilic aromatic substitution reaction, if initially an amino substituted compound of formula IV was deployed. Likewise a compound of formula III in which Z represents aryl or heteroaryl substituted by -NH₂ may be employed in the process of the reaction, but that amino group may be converted to a diazonium salt, and then subsequently to, for example, a -OH group, before or after the process of the reaction.

It is stated herein that specific functional groups may be protected. It will also be appreciated by those skilled in the art that, in the processes described above, other functional groups of intermediate compounds may be, or may need to be, protected by protecting groups.

In any event, functional groups which it is desirable to protect include hydroxy (e.g. R^{a} may represent an oxy-protecting group). Suitable protecting groups for hydroxy include trialkylsilyl and diarylalkyl-silyl groups (e.g. *tert*-butyldimethylsilyl, *tert-*butyldiphenylsilyl or trimethylsilyl), tetrahydropyranyl and alkylcarbonyl groups (e.g. methyl- and ethylcarbonyl groups). However, most preferred protecting groups for hydroxy include alkylaryl groups, such as optionally substituted benzyl.

The protection and deprotection of functional groups may take place before or after any of the reaction steps described hereinbefore.

Protecting groups may be removed in accordance with techniques which are well known to those skilled in the art and as described hereinafter.

The use of protecting groups is described in "Protective Groups in Organic Chemistry", edited by J.W.F. McOmie, Plenum Press (1973), and "Protective Groups in Organic Synthesis", 3rd edition, T.W. Greene & P.G.M. Wutz, Wiley-Interscience (1999).

The skilled person will appreciate that the process of the invention (to obtain a compound of formula I) may proceed *via* an *O*-phenyl oxime intermediate, i.e. a compound of formula XXIV, 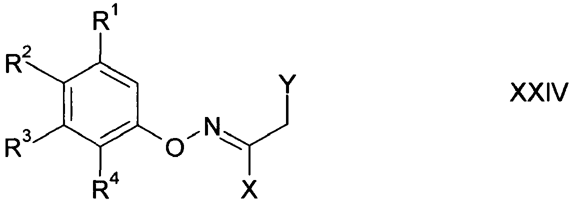 wherein R¹ to R⁴, X and Y are as hereinbefore defined, which intermediate then undergoes a pericyclic rearrangement, ultimately forming a benzofuran ring. It is hereinbefore stated that in an embodiment of the invention, the process of the invention is performed in the absence of an acylating agent. In this instance, when the process of the invention proceeds *via* an intermediate of formula XXIV, then the phenyl oxime intermediate of formula XXIV does not first react with an acylating reagent to form an *N*-acyl group at the imino nitrogen (the relevant imino functional group being converted to enamino functional group), for example as depicted by the following compound of formula XXIVA, 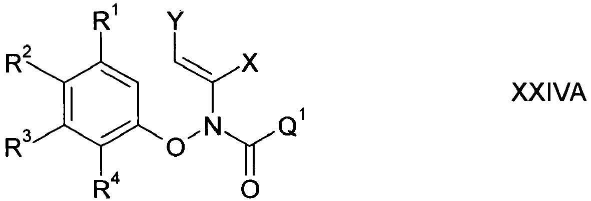 or another enamino equivalent thereof (for example, when X represents an alkyl group, the double bond of the enamino moiety may be adjacent the X group), wherein Q¹ represents, for example, a C₁₋₆ alkyl group optionally substituted by one or more fluoro atoms (so forming, for example a -CF₃ group) and R¹ to R⁴, X and Y are as hereinbefore defined.

Rather, the pericyclic rearrangement of the compound of formula XXIV takes place in the absence of an acylating reagent and hence does not proceed *via* an intermediate of formula XXIVA. Rather, the pericyclic rearrangement is performed under reaction conditions such as those described herein, for example in the presence of acid, such as a weak organic acid as described herein.

Such an intermediate may be separated (e.g. isolated) in the process of the invention and/or reaction conditions may subsequently be modified. That is, in a first reaction step, a compound of formula II may be reacted with a compound of formula III, as hereinbefore defined, to form an intermediate compound of formula XXIV and, in a subsequent reaction step, the intermediate of formula XXIV may undergo reaction (i.e. a pericyclic rearrangement reaction) to form the compound of formula I. Hence, such an embodiment consists of two (e.g. distinct/separate) reaction steps. In such an embodiment, the intermediate compound of formula XXIV may be separated (e.g. extracted, optionally isolated from any impurities, and any solvent optionally removed) from the reaction mixture and/or the subsequent reaction step may be performed under modified reaction conditions (e.g. in the presence of a different, or 'fresh', solvent and/or in the presence of additional reagents).

However, advantageously, any intermediate formed in the process of the present invention (such as an intermediate of formula XXIV) need not be separated and/or reaction conditions need not be modified in order to promote the benzofuran-forming reaction. In essence, therefore, the reaction may be performed as a "one-pot" procedure. Such a "one-pot" procedure is particularly preferred in the case where compounds of formula I in which Y represents H (and/or compounds of formula I in which R² represents -NO₂) are required and/or desired.

Thus, in particular embodiments of the invention, the reaction is performed without separation (e.g. isolation) of any intermediates. In alternative embodiments of the invention, the reaction is conducted without modification of the reaction conditions.

Where it is stated that the reaction is performed without separation of intermediates, we mean that any intermediate that may be formed by reaction of the starting reagents, is not isolated, e.g. in a purified state (whether or not the intermediate is still in the presence of solvent and/or residual starting materials or other impurities). In this context, we therefore include that the any intermediate is not extracted from the reaction of the starting materials. Where it is stated that the reaction conditions need not be modified, we encompass reactions in which the solvent need not be changed and/or that further reagents need not be added.

In yet another aspect of the invention, there is provided a process for the preparation of a compound of formula I as hereinbefore defined, but in which Y represents -C(O)-Z, which comprises reaction, for example an intramolecular reaction (i.e. pericyclic rearrangement), of a compound of formula XXIV in which Y represents -C(O)-Z. Such a reaction may be performed in the absence of an acylating reagent, and may for example be performed under the reaction conditions described herein.

The benzofuran-forming process reaction of the invention is one in which a compound of formula II is reacted with a compound of formula III and is preferably performed in the presence of an acid, such as a weak organic acid (e.g. formic acid or, preferably, acetic acid) and/or an inorganic acid, such as any suitable mineral acid, or suitable salts thereof (for example, nitric acid, sulfuric acid, or salts thereof, such as sodium hydrogen sulphate, or, more preferably, a hydrogen halide acid, e.g. HBr). Mixtures of acids may also be employed, for instance, a mixture of a weak organic acid and an inorganic acid (e.g. HBr and acetic acid). Further, when an acid is employed, then that acid may be a component of an aqueous solution. By *"weak* organic acid", we mean that the organic acid has a pKa (at 25°C) of from 2 to 6 (e.g. from 3 to 5).

The benzofuran-forming process reaction of the invention may be performed in the presence of a suitable solvent, for example water or an organic solvent such as toluene, tetrahydrofuran, diethyl ether, dioxane, dimethylformamide, dimethylsulfoxide, or, preferably an alcohol (such as methanol or ethanol), or mixtures thereof (including biphasic solvent systems, such as a mixture of water and an organic solvent). However, when a weak organic acid is employed (whether it is as the only acid component or as a component of a mixture of acids) in the reaction mixture, then that acid may serve as both the reagent and solvent. In such an instance, advantageously, the separate use of a solvent in the reaction mixture is circumvented (although, as stated above, a mixture of such an organic acid and another suitable solvent, as defined above, may be employed). In particular, weak organic acids that have a relatively low boiling point may serve as the reagent and solvent, for instance those organic acids with a boiling point of less than 150°C (e.g. formic or, more preferably, acetic acid). When, for instance, a weak organic acid (e.g. that serves as reagent and solvent) is employed, then it may be employed as a solution (e.g. in water or an organic solvent) or, e.g. more preferably, it is employed "neat". For instance, when acetic acid is employed, then it may be glacial acetic acid.

When a solvent, or a weak organic acid that serves as a solvent, is employed, then it may be present in any suitable volume. However, it is preferred that the concentration of the compound of formula II in the solvent/weak organic acid solvent is from 0.1 M to 5 M, preferably from 0.5 M to 2 M (e.g. between 0.6 M and 1.5 M).

In the event that the compounds of formula II and III are added to the reaction mixture at the same time, then the concentration of the reagents in the solvents will be higher (in accordance with the molar ratios of the compounds of formulae II and III in the reaction mixture; see below). However, it is preferred that the compound of formula III is added to the compound of formula II (which latter is preferably already in the presence of a solvent or weak organic acid that serves as a solvent), especially when Y represents H in the compound of formula III. However, particularly when Y represents -C(O)-Z in the compound of formula III, then it is particularly preferred that a compound of formula II is added to a compound of formula III (the latter preferably already in the presence of a solvent or weak organic acid that serves as a solvent). Such an order of addition may aid the regioselectivity of the initial intermolecular reaction (for instance, when a compound of formula III in which Y represents -C(O)Z is employed) and/or, in the case where the reaction proceeds *via* an intermediate compound of formula XXIV, this order of addition may also aid the efficiency of the subsequent intramolecular reaction forming the benzofuran ring.

The benzofuran-forming process reaction of the invention may be performed at any suitable reaction temperature, for instance at room or elevated temperature. In certain preferred embodiments of the invention, (e.g. when the reaction takes place in the presence of a mixture of a weak organic acid and strong inorganic acid) the reaction may be performed at room temperature (e.g. for a period of time, such as 6 hours), or, (e.g. when the reaction takes place in the presence of a weak organic acid solvent) the reaction may be performed at elevated temperature (e.g. at above 50°C, such as between 60°C to 80°C) for a period of time (such as 3 hours, or, any suitable period of time up to 25 hours) followed by, if necessary, an increase in reaction temperature (e.g. to at least 80°C, for instance from 90°C to 118°C (e.g. such as 110°C, e.g. 100°C)), for a period of time (such as any suitable period of time up to 25 hours, for instance, 22 hours).

The skilled person will appreciate that the temperature may only be increased up to the boiling point of the solvent system (which may comprise a weak organic acid solvent), for instance, when acetic acid is employed, the reaction temperature may only be increased up to 118°C. Hence, the preferred temperature conditions of the process of the invention are particularly applicable when the process of the reaction is performed in the presence of acetic acid. However, when the benzofuran-forming process reaction is performed in the presence of other weak organic acids (or otherwise another suitable solvent), such as formic acid, the skilled person will appreciate that the preferred reaction temperature conditions referred to herein may be varied, for example in accordance with differing boiling points.

The benzofuran-forming process reaction of the invention may also be conducted under conditions that provide an alternative to typical reaction conditions where elevated temperatures are necessary and/or desired. For instance, microwave irradiation conditions may be employed. By 'microwave irradiation conditions', we include reactions in which such conditions promote a thermally induced reaction (for instance at elevated temperature as hereinbefore described) and/or in which such conditions promote a non-thermally induced reaction (i.e. the reaction is induced by the microwaves). Hence, such reaction conditions are not necessarily accompanied by an increase in temperature. The skilled person will appreciate (and be able to non-inventively determine) that the length of reaction time may be altered (e.g. reduced) when employing such reaction conditions.

The benzofuran-forming process reaction of the invention may also be conducted under pressure, for instance, under a pressure greater than that of normal atmospheric pressure, for example, at a pressure of up to 5 or 6 bars. Again, the skilled person will appreciate (and be able to non-inventively determine) that the length of reaction time may be altered (e.g. appropriately reduced) when employing such reaction conditions.

The benzofuran-forming process reaction of the invention may be performed in the presence of any quantity of each of the compounds of formulae II and III. However, it is preferably performed in the presence of compounds of formulae II and III that are in a molar ratio of from 3:2 to 2:3, and most preferably in a molar ratio of from 1.1:1 to 1:1.1 (e.g. 1:1).

Preferred compounds of formula I that may be prepared by the process of the invention include those in which:
R¹, R², R³ and R⁴ independently represent hydrogen, halo, -NO₂, -CN, -C(O)₂R^{x1}, -N(R^{x6})R^{x7} or -N(R^{x10})S(O)_{2R}^{x11};
X represents C₁₋₆ alkyl;
Z represents heteroaryl or, preferably aryl (e.g. phenyl) optionally substituted by one or more substituents selected from -OR^{a}, -NO₂, -CN, -C(O)₂R^{a1} and -N(R^{a6})R^{a7};
R^{a} represents an oxy-protecting group, hydrogen or C₁₋₄ (e.g. C₁₋₃) alkyl optionally substituted by one or more substituents selected from -N(R^{b2})R^{b3};
R^{x1}, R^{x2}, R^{x3}, R^{x6}, R^{x7}, R^{x8}, R^{x9}, R^{x10}, R^{a1}, R^{a3}, R^{a6}, R^{a7}, R^{a8}, R^{a9}, R^{a10}, R^{b1}, R^{b2} and R^{b3} independently represent hydrogen or C₁₋₄ alkyl optionally substituted by one or more halo atoms;
R^{x4}, R^{x5}, R^{x11}, R^{x12}, R^{a4}, R^{a5}, R^{a11} and R^{a12} independently represent C₁₋₄ alkyl optionally substituted by one or more halo atoms

Further preferred compounds of formula I that may be prepared by the process of the invention include those in which:
any three of R¹, R², R³ and R⁴ (preferably R¹, R³ and R⁴) represent hydrogen; one of R¹, R², R³ and R⁴ (preferably R²) represents a substituent selected from halo, -CN, -C(O)₂R^{x1}, preferably, -N(R^{x10})S(O)₂R^{x11} or, more preferably, -NO₂ or -N(R^{x6})R^{x7};
R^{x1} represents H or C₁₋₃ alkyl (e.g. propyl, such as isopropyl);
R^{x6}, R^{x7} and R^{x10} independently represent hydrogen;
R^{x11} represents C₁₋₂ alkyl (e.g. methyl);
when Z represents phenyl, such a group may be unsubstituted or is preferably substituted, for example by one or two (e.g. one) substitutent(s) in the *ortho* or, preferably in the *para* position;
substituents on Z groups (e.g. when Z represents phenyl) are preferably selected from -CN, -C(O)₂R^{a1}, preferably, -NO₂, -N(R^{a6})R^{a7}, halo (e.g. iodo) and, more preferably, -OR^{a};
R^{a} represents an oxy-protecting group, hydrogen or C₁₋₃ alkyl (e.g. ethyl or, preferably, propyl or methyl) optionally substituted by one or more substituents selected from -N(R^{b2})R^{b3} (so forming, for example a -(CH₂)₂-N(R^{b2})R^{b3} or, preferably, a -(CH₂)₃-N(R^{b2})R^{b3} group);
R^{a1} represents H or C₁₋₃ (e.g. C₁₋₂) alkyl (e.g. propyl, such as isopropyl);
R^{a6} and R^{a7} independently represent hydrogen;
R^{b2} and R^{b3} independently represent H or, preferably, C₁₋₄ alkyl (such as ethyl or preferably butyl, e.g. *n*-butyl).

Further preferred compounds of formula I that may be prepared by the process of the invention include those in which:
R¹, R², R³ and R⁴ independently represent hydrogen or -NO₂;
X represents C₁₋₄ alkyl (e.g. butyl);
Z represents aryl (e.g. phenyl) optionally substituted by one or more substituents selected from halo (e.g. iodo) and, preferably, -OR^{a};
R^{a} represents hydrogen, C₁₋₃ alkyl (e.g. methyl) or an oxy-protecting group (e.g. benzyl).

Particularly preferred compounds of formula I that may be prepared by the process of the invention include those in which:
R¹, R³ and R⁴ independently represent hydrogen;
R² represents -NO₂;
X represents *n*-butyl;
Y represents -C(O)-Z;
Z represents phenyl substituted (e.g. in the *ortho*- or, preferably, in the *para-*position) by one or more (e.g. one) substituent(s) selected from -O-benzyl, -OCH₃ or, more preferably, -OH.

As stated above, it is preferred that compounds of formula I obtained *via* the benzofuran-forming process reaction of the invention are ones in which Y represents -C(O)-Z. Reactions to produce such compounds of formula I (involving reactions of compounds of formula III in which Y represents -C(O)-Z) have the additional advantage that, when 3-aroyl substituted benzofurans are required, a (disadvantageous) Friedel-Crafts acylation step on a 3-unsubstituted benzofuran is circumvented. Further advantages associated with this preferred embodiment of the process of the invention are that compounds of formula I in which Y represents -C(O)-Z may be produced in higher yields as the reaction may proceed in a more regioselective manner than corresponding reactions to produce compounds of formula I in which Y represents H. In this embodiment of the invention, despite the fact that the compound of formula III in which Y represents -C(O)-Z contains two carbonyl moieties, the reaction with the compound of formula II proceeds in a highly regioselective manner, favouring the carbonyl adjacent to (or α- to) the group defined by X (in the initial step condensation reaction between the hydroxylamino moiety of the compound of formula II and the relevant carbonyl group). Surprisingly, this regioselectivity is greater than 90:10 (e.g. 95:5), and selectivities of 99:1 have been achieved.

As stated hereinbefore, it is preferred that compounds of formula I obtained *via* the process of the invention are ones in which R² represents -NO₂. The formation of compounds of formula I in which R² is -NO₂ normally proceeds *via* a reaction of a chlorophenyl group with a hydroxy-imine (e.g. 2-hexanone oxime), which is the conventional manner of performing this reaction.

Further, it is also stated above that particularly preferred compounds of formula I obtained *via* the benzofuran-forming process reaction of the invention are ones in which Z represents phenyl substituted (e.g. in the *para*-position) with -OH. When such compounds of the invention are desired and/or required (for example as an intermediate in the synthesis of Dronedarone), it is particularly advantageous that the process of the invention proceeds when the relevant -OH group is unprotected. For instance, processes described in the prior art (e.g. in US 5,223,510, US 5,854,282 and WO 2007/140989), which relate to the Friedel-Crafts acylation of 3-unsubstituted benzofurans, all result in the formation of 3-(4-methoxybenzoyl)benzofurans. Such intermediates may be employed in the synthesis of Dronedarone, but the methoxy group has to be 'deprotected', i.e. the methyl group has to be cleaved from the methyl aryl ether. Such cleavage conditions may also involve metal halide catalysts, such as group III metal halide catalyst, such as BBr₃ and AlCl₃ (which are disadvantageous in process chemistry for reasons mentioned herein; for example as toxic by-products may be formed, e.g. chloromethane, when AlCl₃ is employed). Hence, given that when compounds of formula I in which Z represents phenyl substituted (e.g. in the para-position) with -OH are prepared, such methyl aryl ether cleavage is circumvented, this embodiment of the invention is particularly preferred. Hence, there are several environmental benefits associated with the process of the invention, and particularly with certain embodiments of the process of the invention.

In a further preferred embodiment of the invention, in the benzofuran-forming process reaction of the invention, a compound of formula II, is reacted with a compound of formula III in which Y represents -C(O)Z, and Z represents an aryl or heteroaryl group (preferably phenyl) substituted (e.g. in the para-position) by a -OR^{a} group, in which R^{a} represents an oxy-protecting group (e.g. benzyl). In this embodiment of the invention, the compound of formula I so formed may be a corresponding one in which R^{a} also represents the oxy-protecting group (e.g. benzyl) or, preferably, one in which R^{a} represents hydrogen (i.e. the deprotected occurs during the process of the invention). Hence, this embodiment of the invention may be particularly preferred as, it may reduce the number of overall (separate) process steps that need to be performed. In such an embodiment an inorganic acid, as hereinbefore defined, may be employed in addition to a weak organic acid as hereinbefore defined.

The compounds of formula I obtained by the process of the invention may be separated and/or isolated by standard techniques, for instance by chromatography, crystallisation, evaporation of solvents and/or by filtration.

Advantageously, the benzofuran-forming process reaction of the invention further comprises the additional step of crystallisation of the compound of formula I from a solution, wherein the solvent is preferably, a non-halogenated solvent. Such a crystallisation may be performed by the addition of a solvent to the reaction mixture of the process of the invention that provides for a compound of formula I (e.g. without prior separation, e.g. isolation, (e.g. by extraction) of the compound of formula I) or, such a crystallisation may be performed after the compound of formula I is separated (e.g. by extraction, optionally followed by removal of solvent) or isolated.

Preferably, the crystallisation mixture/solution (which, in this context, includes a compound of formula I in the reaction mixture after the process of the invention but prior to separation, as well as a compound of formula I that is separated and to which a solvent is then added) is cooled after the addition of the solvent. Conveniently, the mixture is cooled to between -5 and 15°C (for example the optimal temperatures employed are between +5 and 15°C). A preferred 'crystallisation' temperature is -5°C (minus five degrees Celsius). The mixture may be cooled using any suitable means, for example ice-baths or cooling systems well known to those skilled in the art and include, for example, heat exchangers.

The 'crystallisation' solvent may also be used to wash the crystallised product, which solvent is preferably pre-cooled. Possible temperatures to which the solvent may be pre-cooled are between -5°C to 5°C (or, alternatively, the temperature may be between +5 and 15°C). If there is no pre-cooling of the washing solvent, yield may drop. The most preferred temperature is -5°C.

The `crystallisation' solvent is preferably a non-halogenated one, e.g. water or it may be an alcohol, such as methanol ethanol, iso-propanol and 1-propanol. The most preferred 'crystallisation' solvent may be methanol. Other preferred crystallisation solvents that may be mentioned include weak organic acids, for example, carboxylic acids (such as butanoic acid, propanoic acid, preferably, formic acid or, more preferably, acetic acid). Such weak organic acids may be mixed with water to form crystallisation co-solvents. When the crystallisation consists of the addition of solvent to a reaction mixture, then that solvent may be water.

It should be appreciated that the purified compound of formula I so formed by the process of the invention may also contain materials other than those specified above.

This product may be further purified using any suitable separation/purification technique or combination of techniques including further crystallisation, distillation, phase separation, adsorption, e.g. using molecular sieves and/or activated carbon, and scrubbing.

In a further aspect of the invention there is provided a process for preparing Dronedarone: 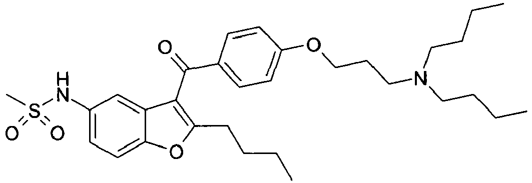 (or a salt, e.g. a hydrochloride salt, thereof), which process is characterised in that it includes as a process step a process as described herein (for instance, a process for the preparation of 2-butyl-3-(4-hydroxybenzoyl)-5-nitrobenzofuran or 2-butyl-3-(4-methoxybenzoyl)-5-nitrobenzofuran).

Hence, there is provided a process for the preparation of Dronedarone, or a salt thereof, comprising a process for the preparation of a compound of formula I (e.g. a process for the preparation of 2-butyl-3-(4-hydroxybenzoyl)-5-nitrobenzofuran or 2-butyl-3-(4-methoxybenzoyl)-5-nitrobenzofuran) as described herein, followed by, if necessary/required:
1) if necessary (i.e. in the case of 2-butyl-3-(4-methoxybenzoyl)-5-nitrobenzofuran), conversion of the "4-methoxy" moiety to a "4-hydroxy" moiety (e.g. by cleavage of the methyl phenyl ether moiety under standard conditions, such as by employing BBrs or AlCl₃); and,
2) conversion of the nitro (-NO₂) group to a methylsulfonylamino (-NHS(O)₂CH₃) group (for example *via* the conversion of the nitro group to an amino (-NH₂) group, followed by reaction with CH₃-S(O)₂-L^{a}, in which L^{a} represent halo, and preferably chloro);
3) conversion of the -OH group to the relevant oxy-alkylaminoalkyl (e.g. -O-(CH₂)₃-N(C₄H₉)₂) group;
4) if necessary/required, conversion of any free base of Dronedarone so formed to a salt (such as a hydrochloride salt).

Such steps are standard steps known to the skilled person, and the steps may be performed in accordance with techniques described in the prior art, such as those references disclosed herein. For example, Dronedarone (or salts thereof) may be prepared from the relevant compounds of formula I using any standard route of synthesising derivatives of benzofuran, such as those described in US 5,223,510. The skilled person will appreciate that the individual steps of the conversions (e.g. those outlined by steps (2) and (3) above) may be performed in any suitable order.

### Step (3)

For example, when the compound of formula I is 2-butyl-3-(4-hydroxybenzoyl)-5-nitrobenzofuran, then such a compound may be reacted as set out by step (3) above, which reaction may be performed in the presence of a compound of formula XXV,

L^{1a1}-(CH₂)₃-N(*n*-butyl)₂ XXV

wherein L^{1a1} is a suitable leaving group, such as a sulfonate group (e.g. a triflate or sulfonate), iodo, bromo or, preferably, chloro, under standard alkylation reaction conditions, for example such as those described in US 5,223,510 (see Example 1 (e)), to form a Dronedarone intermediate compound of formula XXVI,

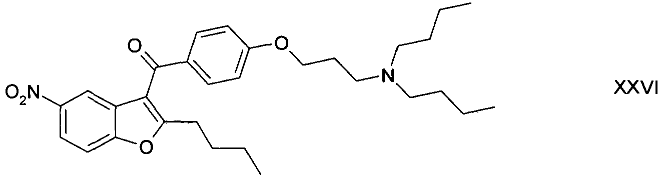

Alternatively, step (3) may be performed in two distinct steps, for example, by reaction of 2-butyl-3-(4-hydroxybenzoyl)-5-nitrobenzofuran with a compound of formula XXVIA,

L^{1a1}-(CH₂)₃-L^{1a1} XXVIA

wherein each L^{1a1} independently represents a suitable leaving group, such as iodo, chloro or, preferably, bromo, so forming a Dronedarone intermediate of formula XXVIB, 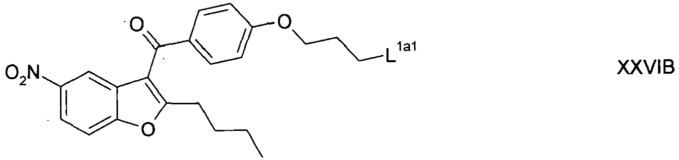 wherein L^{1a1} is as hereinbefore defined (and is preferably bromo), which intermediate may then be reacted with HN(*n*-butyl)₂ (di-*n*-butylamine) to form a Dronedarone intermediate of formula XXVI, for example under reaction conditions such as those described in Chinese patent publication number CN 101153012).

### Step (2)

The intermediate compound of formula XXVI may then be reacted as set out by step (2) above, which may consist of distinct sub-steps:
(i) reduction of the -NO₂ group to a -NH₂ group, under standard reaction conditions, for example such as those described in US 5,223,510 (see Example 1(f)) or in WO 02/48132, for example hydrogenation in the presence of H₂ (e.g. a hydrogen atmosphere or nascent hydrogen, e.g. ammonium formate) and a precious metal catalyst (e.g. PtO₂ or Pd/C), in the presence of an appropriate solvent (e.g. an alcohol, e.g. ethanol), thereby forming an intermediate compound of formula XXVI, 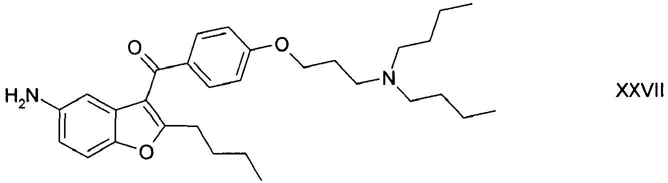
(ii) the Dronedarone intermediate compound of formula XXVII may then be mesylated by reaction with a compound of formula XXVIII,

   H₃C-S(O)₂-L^{1a2} XXVIII

   wherein L^{1a2} represents a suitable leaving group, such as bromo, iodo or, preferably, chloro, under reaction conditions such as those described in US 5,223,510 (Example 3(a)).

### Step 4

As stated above (step (4)), Dronedarone may be converted into a salt, such as a hydrochloride salt, for example as described in US 5,223,510 (see Example 3(b)), for example by bringing into association Dronedarone and HCl in ether, or as described in US 6,828,448 (see Examples, such as Example 4), for example by bringing into association Dronedarone, hydrochloric acid (e.g. 30-40%) and an alcoholic solvent, such as isopropanol.

As stated above the above steps may be performed in any feasible order. Hence, 2-butyl-3-(4-hydroxybenzoyl)-5-nitrobenzofuran may first be reacted as set out in step (2), followed by the reaction(s) as set out in step (3). The preparation of Dronedarone may therefore proceed *via* the following intermediate compounds of formulae XXIX and XXX (step 2), 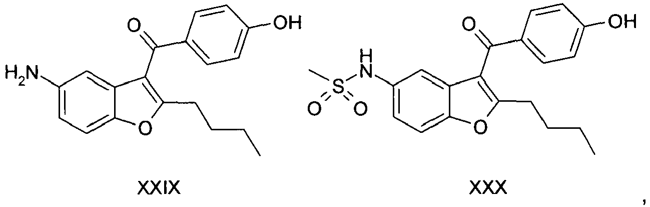 and, may also proceed *via* the intermediate compound of formula XXXI (step (3), when performed as a two-step process), 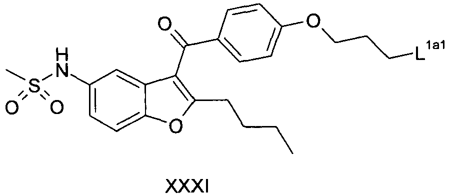 wherein L^{1a1} is as hereinbefore defined.

The skilled person will appreciate that the intermediate compounds of formulae XXVI, XXVIB, XXVII, XXIX, XXX and XXXI may also be compounds of formula I. Hence, the conversion of such compounds of formula I (which may be prepared directly from the process of the invention) may not require all of the process steps (or sub-process steps) outlined above (i.e. steps (1), (2), (3) and (4)) in order to provide Dronedarone, or a salt (e.g. a HCl salt) thereof. In such instance, it is immediately clear to the skilled person which of the above-mentioned steps are required for the appropriate conversions.

There is further provided a process for the preparation of an intermediate of Dronedarone (or a salt thereof, e.g. a hydrochloride salt), which process comprises a process step as hereinbefore described followed by one or more process steps that lead to the formation of Dronedarone, or a salt thereof. For example, such further process steps may include the step (1) outlined above (if necessary/required) and/or any one or more of the process steps disclosed in steps (2), (3) and (4) above, in any feasible order (thereby forming an intermediate of formula XXVI, XXVIB, XXVII, XXIX, XXX or XXXI). The skilled person will appreciate that steps (2), (3) and (4) above may each require multiple separate reaction steps for the relevant conversion to be effected.

The processes described herein may be operated as a batch process or operated as a continuous process and may be conducted on any scale.

In general, the processes described herein, may have the advantage that the compounds of formula I may be produced in a manner that utilises fewer reagents and/or solvents, and/or requires fewer reaction steps (e.g. distinct/separate reaction steps) compared to processes disclosed in the prior art. Processes described herein may also have the advantage that fewer undesired by-products (resultant of undesired side reactions) may be produced, for example, by-products that may be toxic or otherwise dangerous to work with, e.g. explosive.

The processes of the invention may also have the advantage that the compound of formula I is produced in higher yield, in higher purity, in higher selectivity (e.g. higher regioselectivity), in less time, in a more convenient (i.e. easy to handle) form, from more convenient (i.e. easy to handle) precursors, at a lower cost and/or with less usage and/or wastage of materials (including reagents and solvents) compared to the procedures disclosed in the prior art. Furthermore, there may be several environmental benefits of the process of the invention, such as the circumvention of the use of halogenated solvents (e.g. when avoiding the need to perform a Friedel-Crafts reaction or a deprotection of e.g. a -OCH₃ group, which may be required for certain steps performed by processes in the prior art, to a -OH group).

The following examples and reference examples are merely illustrative examples of the processes of the invention and other processes described herein.

All equipment, reagents and solvents used were standard laboratory equipment, e.g. glassware, heating apparatus and HPLC apparatus.

### EXAMPLE 1

### 0-4-Nitrophenyl hydroxylamine,

240 g Water-moist Ethyl-N-(4-nitrophenoxy) acetimidate, containing 181 g, 0.807 mol of product (when dry) was added to 397g 37 % hydrochloric acid (5eq) in portions over 50 minutes, keeping the temperature at 25-32°C. Analysis (HPLC) after 60 minutes showed a conversion of 99.9%. The slurry was diluted with 37 ml water and then neutralised with 580g 33% NaOH keeping the temperature below 33°C. The slurry was then cooled to 24°C, filtered, and the filter cake washed with 210 ml water. Drying afforded 124.5g 0-4-nitrophenyl hydroxylamine. Assay (NMR) 99.8 %, chromatographic purity (HPLC) 99.4 area %. Yield 99.9 %

### EXAMPLE 2

### Method A

### 2-Butyl-3-(4-hydroxybenzoyl)-5-nitrobenzofuran

(a) 4-Benzyloxy acetophenone (10 g) and ethyl pentanoate (1.2 equiv.) were dissolved in toluene (30 g) containing DMF (6.5 g). The mixture was heated to 65°C and NaOMe (3 eq) was added in portions over 3.5 h. Analysis of a sample withdrawn after 4 h showed a conversion of 97 %. The reaction mixture was quenched by addition to water (30 ml). This was proceeded by acidification with hydrochloric acid and extraction with toluene (40 ml), followed by solvent change to MeOH (100 ml). The product, which crystallises upon cooling, was collected by filtration, washed with methanol and dried under vacuum. Yield 8.04 g of 1-(4-benzyloxyphenyl)-heptane-1,3-dione.
(b) 1-(4-Benzyloxyphenyl)-heptane-1,3-dione (4 g; see step (a) above) was dissolved in toluene (20 ml) and Pd/C (3%; 80 mg) was added. The mixture was stirred at room temperature until hydrogen uptake ceased. After filtration of the catalyst, the solvent was evaporated leaving 2.84 g, 100 %, 1-(4-hydroxyphenyl)-heptane-1,3-dione.
(c) 0-4-nitrophenylhydroxylamine prepared according to Example 1 (1.0 g), was suspended in acetic acid (10 ml) and 1-(4-hydroxyphenyl)-heptane-1,3-dione (1.36 g; see step (b) above) was added. The mixture was stirred for 3h at 70°C and then at 100°C for an additional 22h. The mixture was cooled to room temperature and the solvent evaporated under vacuum. Yield 80 % of 2-Butyl-3-(4-hydroxybenzoyl)-5-nitrobenzofuran.

### Method B

### 2-Butyl-3-(4-hydroxybenzoyl)-5-nitrobenzofuran

1-(4-Benzyloxyphenyl)-heptane-1,3-dione (191 mg; see Example 1 (a)), was suspended in 1ml HBr/acetic acid and O-4-nitrophenylhydroxylamine (prepared according to Example 1), 100 mg, was added. The mixture was stirred at room temperature for 6h. After quenching with water and extraction to EtOAc followed by evaporation of the solvent, a crude material containing approximately 125 mg of the title compound was obtained. Yield *ca.* 59 %.

### Method C

### 2-Butyl-3-(4-methoxybenzoyl)-5-nitrobenzofuran

*O*-4-nitrophenylhydroxylamine prepared according to Example 1 (100 mg), was suspended in 0.5 ml acetic acid and 1-(4-methoxyphenyl)-heptane-1,3-dione was added. The mixture was stirred at 70°C for 3h and then at 100°C for an additional 14h. The mixture was cooled to room temperature and the solvent evaporated under vacuum. Yield 70 % of 2-butyl-3-(4-methoxybenzoyl)-5-nitrobenzofuran.

### Method D

### Synthesis of Dronedarone

Dronedarone is synthesised using standard synthetic processes described in the prior art (and referenced herein) incorporating any of the processes described herein, for example the processes to the intermediates 2-butyl-3-(4-hydroxybenzoyl)-5-nitrobenzofuran and 2-butyl-3-(4-methoxybenzoyl)-5-nitrobenzofuran described in Example 2 (Methods A, B and C above). Dronedarone can be made from these intermediates using any standard routes for converting a nitro (-NO₂) group to a methylsulfonylamino (-NHS(O)₂CH₃) group (for example *via* an amino (-NH₂) group) and converting a -OH (or -OCH₃) group to any relevant oxy-alkylaminoalkyl (e.g. -O-(CH₂)₃-N(C₄H₉)₂) group. Further, salts (such as hydrochloride salts) of the relevant compounds may also be prepared. Such steps are standard steps known to the skilled person, and the steps may be performed in accordance with techniques described in the prior art, such as those references disclosed herein.

### EXAMPLE 3

### Method A

### Ethyl N-(4-nitrophenoxy)acetimidate

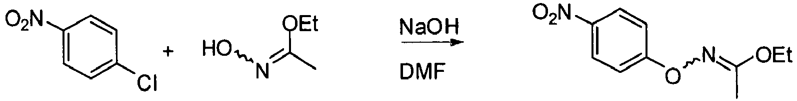

4-Chloronitrobenzene, 136.2g, and 111.4g ethyl N-hydroxyacetimidate are dissolved in 216 ml DMF. The temperature is adjusted to 30°C and 41.6g solid NaOH is added in 8 portions keeping the temperature at 30-35°C. After one hour the temperature is adjusted to 40-45°C and the mixture stirred for 1.5 hours. Cooling is applied and 520 ml water is fed at such a rate as to keep the temperature at ca 40°C. The slurry formed is cooled to 17°C and filtered. The filter cake is washed with 175 ml ethanol/water 90/10 (V/V) followed by 175 ml water. Wet product, 214.5 g, corresponding to 192 g dry ethyl N-(4-nitrophenoxy)acetimidate is isolated. Yield 98.5 %.

### Method B

### Ethyl N-(4-nitrophenoxy)acetimidate

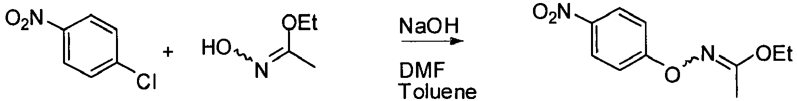

To a solution of 549g ethyl N-hydroxy acetimidate in 976g toluene is added 1267g DMF, 39.9g Aliquat 336 and 799g 4-chloronitrobenzene. The temperature is adjusted to 30°C and 223g solid NaOH is added in portions of 25-30g every 10-15 minutes. When addition is complete, the jacket temperature is set to 40°C and the mixture stirred until reaction is complete, 3-4 h. The jacket temperature is adjusted to 50°C and ca 80 % of the toluene stripped at reduced pressure. 3040g Water is added keeping the temperature at max 45°C. The formed slurry is efficiently agitated and the residual toluene stripped at reduced pressure. After cooling to 15°C. the product is filtered and washed with 1080g EtOH/water 90/10 (V/V) followed by 1080g water. Wet product, 1188 g, corresponding to 1080g dry ethyl N-(4-nitrophenoxy)acetimidate is obtained. Yield 95 %.

### Method C

### O-(4-Nitrophenyl)hydroxylamine

This compound was prepared in accordance with Example 1 described above.

### Method D

### (a) 1-(4-Hydroxyphenyl)-1,3-heptandione

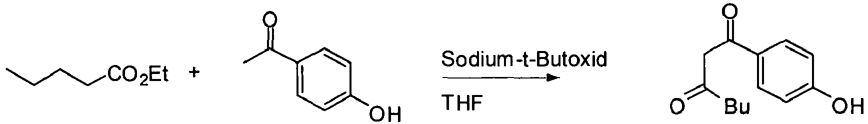

Sodium tert-butoxide, 1270g, is slurried in 1390g THF and the mixture heated to reflux temperature. A solution of 580g 4-hydroxyacetophenone and 555g ethylvalerate in 1390g THF is added over 30 minutes. The solution is stirred at reflux temperature until the reaction is complete, ca 4.5 h, and then quenched by addition of the reaction mixture to 1270g 37 % HCl. The mixture is concentrated by distillation of THF at reduced pressure and to the residue is added 900g toluene. The water phase is separated and the toluene phase washed with 900g 10 % aqueous NaCl. The toluene is stripped at reduced pressure and the residual oil diluted with 850g acetic acid. The solution is cooled to 8°C and 850ml water added slowly. The formed slurry is stirred at 5-8°C for 90 minutes and then filtered and washed with 608g 20% aqueous acetic acid. Drying under vacuum at 40°C gives 608g 1-(4-hydroxyphenyl)-1,3-heptandione. Yield 65 %

### (b) 1-(4-Hydroxyphenyl)-1,3-heptandione

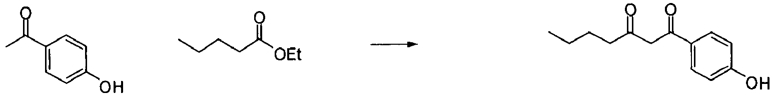

Sodium t-butoxide, 180.5g, 1.878 mol, is mixed and stirred with 378 ml THF. A mixture of 4-hydroxy acetophenone, 85.3g, 0.626 mol and ethyl valerate, 81.5g, 0.626 mol in 56 ml THF is heated to ca 45°C and the clear solution is added to the sodium t-butoxide/THF mixture. The mixture is heated to reflux temperature (ca 68°C) and stirred for 6h. The temperature is adjusted to ca 60°C and the viscous mixture is quenched by addition to a solution of 120g acetic acid in 294 ml water. THF and other volatiles are stripped and the residual emulsion is extracted with 146 ml toluene. After separation of the water phase, the residue is concentrated under vacuum and the product crystallised from a mixture of 130 ml acetic acid and 138 ml water. The product is isolated by filtration and the filter cake washed with 20 % acetic acid followed by water. The wet product is dried under vacuum to afford 93.1g, 0.423 mol 1-(4-hydroxyphenyl) heptane-1,3-dione. Yield 67.5 %.

### (c) 1-(4-Hydroxyphenyl)-1,3-heptandione

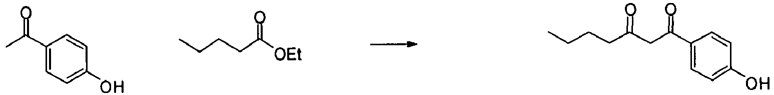

To a solution of 4-hydroxy acetophenone, 13.6g, 0.10 mol, in 74 ml ethyl valerate. is added sodium tert-butoxide, 29.7g, 0.31 mol, in portions. The formed slurry is heated to 82°C and stirred for 4 hours after which the mixture is quenched by addition to a solution of 2 ml acetic acid in 47 ml water. The product-containing lower water phase is separated and treated with acetic acid, 16 ml, to reach pH 4. The upper oily phase is separated and diluted with 20 ml acetic acid and 2.3 g water. The mixture is cooled and crystals starts to separate at 20°C. Cooling is continued to 5°C. 19 ml Water is added over 25 minutes followed by stirring for 20 minutes and then the product is isolated by filtration, washed with 23.5 g 20 % acetic acid followed by 23.5 g water. Drying at room temperature in an air stream afforded 14.6 g 1-(4-hydroxyphenyl)heptane-1,3-dione. Purity (HPLC) < 99.8 %, yield 65 %. The upper phase from the quench is diluted with 30 ml toluene and a small water phase is separated. Concentration of the organic phase followed by distillation afforded crude ethyl valerate, 48 % of theoretic recovery.

### Method E

### 2-Butyl-3-(4-hydroxybenzoyl)-5-nitrobenzofuran

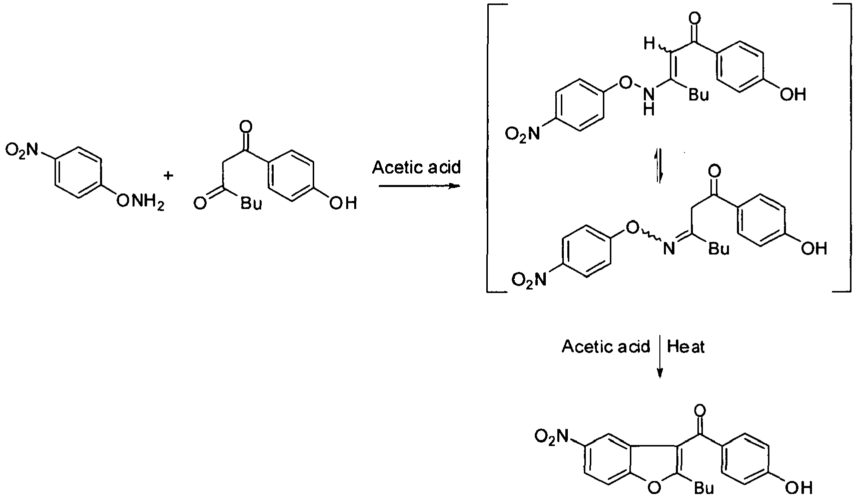

1-(4-hydroxyphenyl)-1,3-heptandione (see Method D, reactions (a), (b) and/or (c)), 697g, is dissolved in 2532g acetic acid. *O*-(4-Nitrophenyl)hydroxylamine (prepared in accordance with Example 1), 488g, is added in portions at ca 20°C. The formed slurry is diluted with 739g acetic acid and the mixture heated to 115°C and stirred for 3h. The dark solution is cooled and 1635g water is added keeping the temperature at 70-80°C. The temperature is adjusted to 60°C and seeding crystals are added. When crystallisation has started, the slurry is cooled to 4°C, filtered and washed with 870g of 67 % aqueous acetic acid followed by 580g water. Drying at reduced pressure at 70°C gives 736g 2-butyl-3-(4-hydroxybenzoyl)-5-nitrobenzofuran. Yield 69 %.

### Method F

### 1-(4-Hydroxyphenyl)heptane-1,3-dione-3-[O-(4-nitrophenyl)oxime]

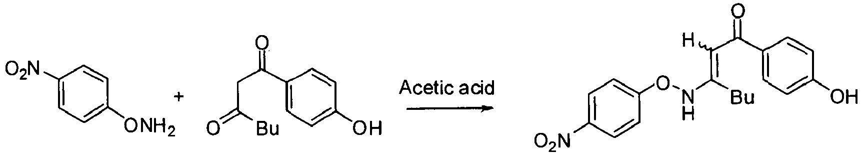

1-(4-Hydroxyphenyl)-1,3-heptandione (see Method D, reactions (a), (b) and/or (c)), 1121g, is dissolved in 4070g acetic acid. *O*-(4-Nitrophenyl)hydroxylamine, 784g, is added in portions keeping the temperature at ca 20°C. The formed slurry is stirred for 3h, cooled to 15°C, filtered and washed with 1590g acetic acid. 1944g wet cake corresponding to 1596g dry 1-(4-hydroxyphenyl)heptane-1,3-dione-3-[O-(4-nitrophenyl)oxime] is obtained. Yield 88 %.

### Method G

### 2-Butyl-3-(4-hydroxybenzoyl)-5-nitrobenzofuran.

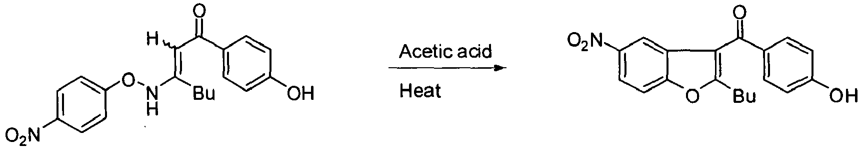

The wet 1-(4-hydroxyphenyl)heptane-1,3-dione-3-[O-(4-nitrophenyl)oxime], 1944g, obtained in Method F is slurried in 4900g acetic acid. The slurry is heated to 115°C and stirred for 3h. The dark solution formed is cooled and 2630g water is added keeping the temperature at 70-80°C. The temperature is adjusted to 60°C and seeding crystals are added. When crystallisation has started, the slurry is cooled to 4°C, filtered and washed with 1400g of 67 % aqueous acetic acid followed by 930g water. Drying at reduced pressure at 70°C gives 1182g 2-butyl-3-(4-hydroxybenzoyl)-5-nitrobenzofuran. Yield 78 %.

### Method H - Synthesis of Dronedarone

Dronedarone is synthesised using standard synthetic processes described in the prior art (and referenced herein) incorporating any of the processes described herein, for example the processes to the intermediates 2-butyl-3-(4-hydroxybenzoyl)-5-nitrobenzofuran and 2-butyl-3-(4-methoxybenzoyl)-5-nitrobenzofuran described in Example 3 above (Methods A to H). Dronedarone can be made from these intermediates using any standard routes for converting a nitro (-NO₂) group to a methylsulfonylamino (-NHS(O)₂CH₃) group (for example *via* an amino (-NH₂) group) and converting a -OH (or -OCH₃) group to any relevant oxy-alkylaminoalkyl (e.g. -O-(CH₂)₃-N(C₄H₉)₂) group. Further, salts (such as hydrochloride salts) of the relevant compounds may also be prepared. Such steps are standard steps known to the skilled person, and the steps may be performed in accordance with techniques described in the prior art, such as those references disclosed herein.

### EXAMPLE 4

Dronedarone may be formulated into a pharmaceutically acceptable formulation using standard procedures, for example to form the product marketed under the brand name, Multaq®.

For example, there is provided a process for preparing a pharmaceutical formulation comprising Dronedarone, or a salt thereof (e.g. a hydrochloride salt), which process is characterised in that it includes as a process step a process as hereinbefore defined. The skilled person will know what such pharmaceutical formulations will comprise/consist of (e.g. a mixture of active ingredient (i.e. Dronedarone or a salt thereof) and pharmaceutically acceptable excipient, adjuvant, diluent and/or carrier).

There is further provided a process for the preparation of a pharmaceutical formulation comprising Dronedarone (or a salt thereof, e.g. a hydrochloride salt; which formulation may be Multaq®), which process comprises bringing into association Dronedarone, or a pharmaceutically acceptable salt thereof (which may be formed by a process as hereinbefore described), with (a) pharmaceutically acceptable excipient(s), adjuvant(s), diluent(s) and/or carrier(s).

There is further provided a process for the preparation of a pharmaceutical formulation comprising Dronedarone (or a salt thereof, e.g. a hydrochloride salt) as described in the art (for example in US 5,985,915 (see Example 3), US 2004/0044070 (see Examples 1 to 5), US 7,323,439, US 2008/0139645 and/or CN 101152154), which process comprises bringing into association Dronedarone (or a salt thereof, e.g. a hydrochloride salt), with the other ingredients of the relevant formulations. For example, Dronedarone hydrochloride may be brought into association with: maize starch, talc, anhydrous colloidal silica, magnesium stearate and lactose (see Example 3 of US 5,985,915); mannitol, anhydrous sodium dihydrogen phosphate and, optionally, water (see Example 5 of US 5,985,915); hydroxypropyl-β-cyclodextrin, monosodium phosphate dehydrate and mannitol (see Example 1 of US 2004/0044070); hydroxypropyl-β-cyclodextrin, anhydrous sodium dihydrogen phosphate, mannitol and, optionally, water (see Examples 2 and 3 of US 2004/0044070); mixture of methylated derivatives of β-cyclodextrin, mannitol and, optionally, water (see Example 4 of US 2004/0044070). The formulations described may be oral tablet forms or injectable forms (e.g. US 2004/0044070 may describe injectable forms).

In particular, there may be further provided a process for the preparation of a pharmaceutical formulation, comprising bringing into association Dronedarone (or a salt thereof; prepared in accordance with the processes described herein), with a pharmaceutically acceptable non-ionic hydrophilic surfactant selected from poloxamers (e.g. poloxamer 407; Synperonic® PE/F127), optionally in combination with one or more pharmaceutical excipients, for example as described in US 7,323,493. For example, Dronedarone hydrochloride may be brought into association with: methylhydroxypropylcellulose, lactose monohydrate, modified corn starch, polyvinylpyrrolidone, Synperonic® PE/F127 and, optionally, any one or more of anhydrous colloidal silica, magnesium stearate and water (see e.g. Tablet A and Examples 1 to 3 of US 7,323,493); modified corn starch, lactose monohydrate, talc, anhydrous colloidal silica and magnesium stearate (see e.g. gelatin capsule of US 7,323,493); microcrystalline cellulose, anhydrous colloidal silica, anhydrous lactose, polyvinylpyrrolidone, Synperonic® PE/F127 and, optionally, one or more of macrogol 6000 and magnesium stearate (see Examples 4 to 6 of US 7,323,493); microcrystalline cellulose, corn starch, polyvinylpyrrolidone, Synperonic® PE/F127, anhydrous colloidal silica, magnesium stearate and lactose monohydrate (see Examples 7 and 8 of US 7,323,493). The skilled person will appreciate that for example in the above-mentioned list of ingredients, every single ingredient need not be present in the formulation (and hence, the process for preparing the formulation may comprise bringing Dronedarone into association with only some of the ingredients mentioned above). Further, where an ingredient is mentioned, the skilled person will appreciate that it may be replaced by another equivalent or similar ingredient that serves the same function (for example Synperonic® PE/F127 may be replaced by another suitable surfactant and methylhydroxypropylcellulose and corn starch may be replaced by another ingredient, such as a suitable disintegrating agent or bioadhesion promoting agent).

## Claims

1. A process for the preparation of a compound of formula I, 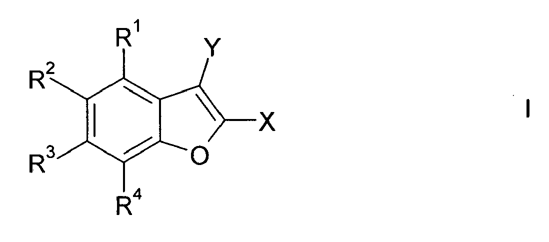 wherein R¹, R², R³ and R⁴ independently represent hydrogen, halo, -NO₂, -CN, -C(O)₂R^{x1}, -OR^{x2}, -SR^{x3}, -S(O)R^{x4}, -S(O)₂R^{x5}, -N(R^{x6})R^{x7}, -N(R^{x8})C(O)R^{x9}, -N(R^{x10})S(O)₂R^{x11} or R^{x12};
X represents hydrogen or C₁₋₆ alkyl optionally substituted by one or more halo atoms;
Y represents -C(O)-Z;
Z represents aryl or heteroaryl, both of which are optionally substituted by one or more substituents selected from -OR^{a}, halo, -NO₂, -CN, -C(O)₂R^{a1}, -SR^{a3}, -S(O)R^{a4}, -S(O)₂R^{a5}, -N(R^{a6})R^{a7}, -N(R^{a8})C(O)R^{a9}, -N(R^{a10})S(O)₂R^{a11} and R^{a12};
R^{a} represents an oxy-protecting group, hydrogen or C₁₋₆ alkyl optionally substituted by one or more substituents selected from halo, -C(O)₂R^{b1} and -N(R^{b2})R^{b3};
R^{x1}, R^{x2}, R^{x3}, R^{x6}, R^{x7}, R^{x8}, R^{x9}, R^{x10}, R^{a1}, R^{a3}, R^{a6}, R^{a7}, R^{a8}, R^{a9}, R^{a10}, R^{b1}, R^{b2} and R^{b3} independently represent hydrogen or C₁₋₆ alkyl optionally substituted by one or more halo atoms;
R^{x4}, R^{x5}, R^{x11}, R^{x12}, R^{a4}, R^{a5}, R^{a11} and R^{a12} independently represent C₁₋₆ alkyl optionally substituted by one or more halo atoms;
which process comprises reaction of a compound of formula II, 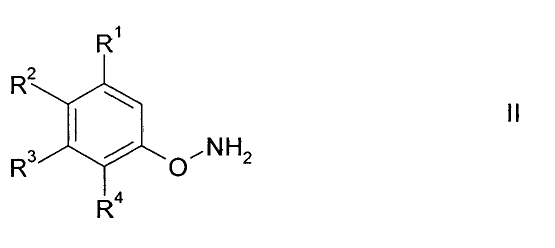 wherein:
R¹, R², R³ and R⁴ independently represent hydrogen, halo, -NO₂, -CN, -C(O)₂R^{x1}, -OR^{x2}, -SR^{x3}, -S(O)R^{x4}, -S(O)₂R^{x5}, -N(R^{x6})R^{x7}, -N(R^{x8})C(O)R^{x9}, -N(R^{x10})S(O)₂R^{x11} or R^{x12};
R^{x1}, R^{x2}, R^{x3}, R^{x6}, R^{x7}, R^{x8}, R^{x9} and R^{x10} independently represent hydrogen or C₁₋₆ alkyl optionally substituted by one or more halo atoms;
R^{x4}, R^{x5}, R^{x11} and R^{x12} independently represent C₁₋₆ alkyl optionally substituted by one or more halo atoms;
with a compound of formula III, 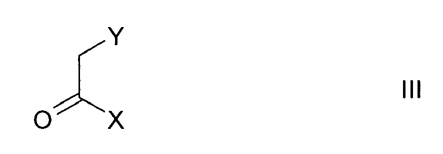
wherein Y and X are as defined above,
and wherein the process further comprises preparation of the compound of formula II *via* a process comprising deprotection of a compound of formula IIA, 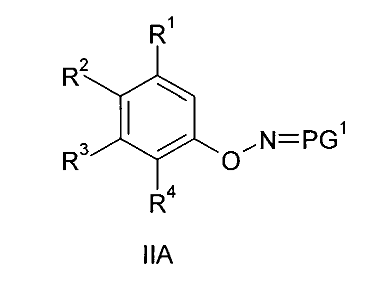
wherein:
PG¹ represents an imino-protecting group;
and R¹, R², R³ and R⁴ are as defined above,
**characterised in that** the deprotection of a compound of formula IIA is performed in the presence of a hydrogen halide, phosphoric acid or sulfuric acid and a solvent system comprising at least 25% by weight of water.

2. A process as claimed in Claim 1, wherein the solvent system is one in which water is present in a molar ratio of at least 5:1 of water:organic solvent.

3. A process for the preparation of a compound of formula I as claimed in Claim 1, but **characterised in that**:
(a) the reaction is performed as a "one-pot" procedure;
(b) R² represents -NO₂; and/or
(c) the process is performed in the absence of an acylating reagent.

4. A process as claimed in any one of the preceding claims, wherein: X represents n-butyl; and/or Z represents phenyl substituted in the para-position by -OH, -OCH₃ or -O-benzyl.

5. A process as claimed in any one of the preceding claims, wherein the reaction used in the preparation of a compound of formula I is performed in the presence of an acid.

6. A process as claimed in Claim 5, wherein the acid is a weak organic acid.

7. A process as claimed in Claim 6, wherein the concentration of the compound of formula II in the weak organic acid solvent is from 0.1 M to 5 M.

8. A process as claimed in any one of Claims 1 to 7, wherein:
(I) the compound of formula II is added to the compound of formula III;
(II) the reaction used in the preparation of a compound of formula I is performed at elevated temperature; and/or
(III) the presence of compounds of formulae II and III are in a molar ratio of from 3:2 to 2:3.

9. A process as claimed in Claim 1, wherein in respect of the preparation of a compound of formula II:
(i) the reaction is performed in the presence of a hydrogen halide;
(ii) the solvent system comprises at least 50% by weight of water;
(iii) any three of R¹, R², R³ and R⁴ represent hydrogen; or
(iv) any one of R¹, R², R³ and R⁴ represents -NO₂.

10. A process as claimed in Claim 1, wherein in respect of the preparation of a compound of formula II:
(A) the hydrogen halide is HCl; or
(B) wherein the solvent system consists of water.

11. A process as claimed in any one of the preceding claims, wherein:
(a) the compound of formula IIA is added to the acid;
(b) PG¹ represents =C(R^{q1})OR^{q2}, in which R^{q1} and R^{q2} independently represent C₁₋₆ alkyl; and/or
(c) the process step further comprises neutralisation to obtain a free base of a compound of formula II.

12. A process as claimed in any one of Claims 1 to 11, wherein the process proceeds *via* an intermediate of formula XXIV, 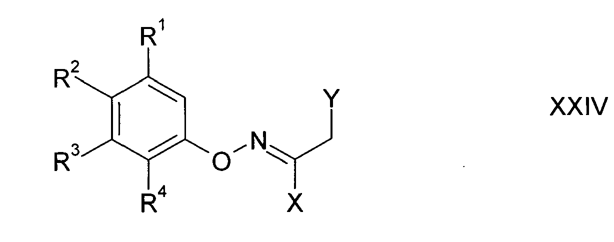 in which Y, R¹, R², R³, R⁴, X and Z are as defined in any one of Claims 1, 3 or 4.

13. A process as claimed in any one of Claims 1 to 12, wherein the process further comprises the additional step of crystallisation of the compound of formula I from a solution.

14. A process for preparing:
(I) Dronedarone, or a salt thereof; or
(II) a pharmaceutical formulation comprising Dronedarone, or a salt thereof,
which process is **characterised in that** it includes as a process step a process as claimed in any one of Claims 1 to 13.

15. A process for the preparation of Dronedarone, or a salt thereof, as claimed in Claim 14, which comprises:
1) a process for the preparation of 2-butyl-3-(4-hydroxybenzoyl)-5-nitrobenzofuran or 2-butyl-3-(4-methoxybenzoyl)-5-nitrobenzofuran as claimed in any one of Claims 1 to 14 ;
2) in the case of 2-butyl-3-(4-methoxybenzoyl)-5-nitrobenzofuran), conversion of the 4-methoxy moiety to a 4-hydroxy moiety; followed by, in any feasible order,
3) conversion of the nitro (-NO₂) group to a methylsulfonylamino (-NHS(O)₂CH₃) group;
4) conversion of the -OH group to the -O-(CH₂)₃-N(C₄H₉)₂ group; and
5) if necessary/required, conversion of any free base of Dronedarone so formed to a salt.

16. A process as claimed in Claim 15, wherein step (1) comprises the preparation of 2-butyl-3-(4-hydroxybenzoyl)-5-nitrobenzofuran, which is followed by step (4), then step (3), then step (5).

17. A process for the preparation of a pharmaceutical formulation comprising Dronedarone, or a salt thereof, which process comprises a process for the preparation of Dronedarone, or, a salt thereof, as claimed in Claim 14, 15 or 16, followed by bringing into association Dronedarone or a salt thereof so formed, with
(i) (a) pharmaceutically-acceptable excipient(s), adjuvant(s), diluent(s) or carrier(s); or
(ii) a pharmaceutically acceptable non-ionic hydrophilic surfactant selected from poloxamers, and, optionally, one or more pharmaceutical excipients.

18. A process for the preparation of an intermediate of Dronedarone, or a salt thereof, which process comprises a process step as claimed in any one of Claims 1 to 13, followed by any one or more process steps disclosed in (1), (2), (3) and (4) described in Claim 15.

## Patentansprüche

1. Verfahren zum Herstellen einer Verbindung der Formel I, 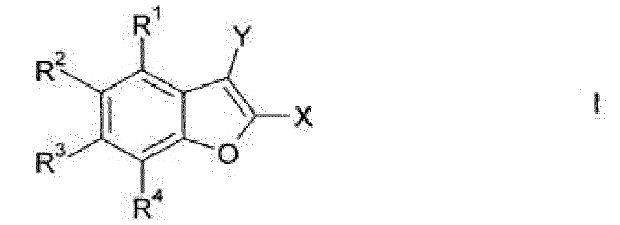 wobei R¹, R², R³ und R⁴ unabhängig Wasserstoff, Halo, -NO₂, -CN, -C(O)₂R^{x1}, -OR^{x2}, -SR^{x3}, - S(O)R^{x4}, -S(O)₂R^{x5}, -N(R^{x6})R^{x7}, -N(R^{x8})C(O)R^{x9}, -N(R^{x10})S(O)₂R^{x11} oder R^{x12} darstellen;
X Wasserstoff oder C₁₋₆-Alkyl, optional mit einem oder mehreren Halo-Atomen substituiert, darstellt;
Y -C(O)-Z darstellt;
Z Aryl oder Heteroaryl darstellt, die beide optional mit einem oder mehreren Substituenten, ausgewählt aus -OR^{a}, Halo, -NO₂, -CN, -C(O)₂R^{a1}, -SR^{a3}, -S(O)R^{a4}, -S(O)₂R^{a5}, -N(R^{a6})R^{a7}, - N(R^{a8})C(O)R^{a9}, -N(R^{a10})S(O)₂R^{a11} und R^{a12}, substituiert sind;
R^{a} eine Oxy-Schutzgruppe, Wasserstoff oder C₁₋₆-Alkyl, optional mit einem oder mehreren Substituenten, ausgewählt aus Halo, -C(O)₂R^{b1} und -N(R^{b2})R^{b3}, substituiert, darstellt; R^{x1}, R^{x2}, R^{x3}, R^{x6}, R^{x7}, R^{x8}, R^{x9}, R^{x10}, R^{a1}, R^{a3}, R^{a6}, R^{a7}, R^{a8}, R^{a9}, R^{a10} R^{b1}, R^{b2} und R^{b3} unabhängig Wasserstoff oder C₁₋₆-Alkyl, optional mit einem oder mehreren Halo-Atomen substituiert, darstellen;
R^{x4}, R^{x5}, R^{x11} R^{x12}, R^{a4}, R^{a5}, R^{a11} und R^{a12} unabhängig C₁₋₆-Alkyl, optional mit einem oder mehreren Halo-Atomen substituiert, darstellen;
wobei das Verfahren umfasst: die Reaktion einer Verbindung der Formel II, 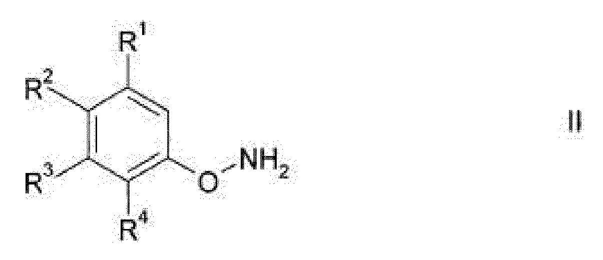 wobei:
R¹, R², R³ und R⁴ unabhängig Wasserstoff, Halo, -NO₂, -CN, -C(O)₂R^{x1}, -OR^{x2}, -SR^{x3}, - S(O)R^{x4}, -S(O)₂R^{x5}, -N(R^{x6})R^{x7}, -N(R^{x8})C(O)R^{x9}, -N(R^{x10})S(O)₂R^{x11} oder R^{x12} darstellen;
R^{x1}, R^{x2}, R^{x3}, R^{x6}, R^{x7}, R^{x8}, R^{x9} und R^{x10} unabhängig Wasserstoff oder C₁₋₆-Alkyl, optional mit einem oder mehreren Halo-Atomen substituiert, darstellen;
R^{x4}, R^{x5}, R^{x11} und R^{x12} unabhängig C₁₋₆-Alkyl, optional mit einem oder mehreren Halo-Atomen substituiert, darstellen;
mit einer Verbindung der Formel III, 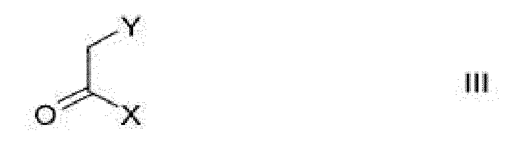
wobei Y und X der vorstehenden Definition entsprechen,
und wobei das Verfahren weiterhin umfasst: das Herstellen der Verbindung der Formel II über ein Verfahren, welches das Entschützen einer Verbindung der Formel IIA umfasst, 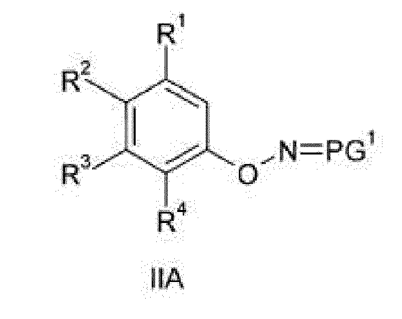
wobei:
PG¹ eine Imino-Schutzgruppe darstellt;
und R¹, R², R³ und R⁴ der vorstehenden Definition entsprechen,
**dadurch gekennzeichnet, dass** das Entschützen einer Verbindung der Formel IIA in Gegenwart eines Wasserstoffhalogenids, von Phosphorsäure oder Schwefelsäure und eines wenigstens 25 Gew.-% Wasser umfassenden Lösungsmittelsystems erfolgt.

2. Verfahren nach Anspruch 1, wobei das Lösungsmittelsystem eines ist, in dem Wasser mit einem Molverhältnis von Wasser zu organischem Lösungsmittel von wenigstens 5:1 vorhanden ist.

3. Verfahren zum Herstellen einer Verbindung der Formel I nach Anspruch 1, jedoch **dadurch gekennzeichnet, dass**:
(a) die Reaktion als "Eintopfverfahren" durchgeführt wird;
(b) R² -NO₂ darstellt; und/oder
(c) das Verfahren in Abwesenheit eines Acylierungsmittels durchgeführt wird.

4. Verfahren nach einem der vorangehenden Ansprüche, wobei: X *n*-Butyl darstellt; und/oder Z Phenyl darstellt, das an der *para*-Position mit -OH, -OCH₃ oder -O-Benzyl substituiert ist.

5. Verfahren nach einem der vorangehenden Ansprüche, wobei die bei der Herstellung einer Verbindung der Formel I verwendete Reaktion in Gegenwart einer Säure durchgeführt wird.

6. Verfahren nach Anspruch 5, wobei die Säure eine schwache organische Säure ist.

7. Verfahren nach Anspruch 6, wobei die Konzentration der Verbindung der Formel II in dem Lösungsmittel, das eine schwache organische Säure ist, 0,1 M bis 5 M beträgt.

8. Verfahren nach einem der Ansprüche 1 bis 7, wobei:
(I) die Verbindung der Formel II zu der Verbindung der Formel III zugegeben wird;
(II) die bei der Herstellung einer Verbindung der Formel I verwendete Reaktion bei erhöhter Temperatur durchgeführt wird; und/oder
(III) Verbindungen der Formeln II und III mit einem Molverhältnis von 3:2 bis 2:3 vorhanden sind.

9. Verfahren nach Anspruch 1, wobei bezüglich der Herstellung einer Verbindung der Formel II:
(i) die Reaktion in Gegenwart eines Wasserstoffhalogenids durchgeführt wird;
(ii) das Lösungsmittelsystem wenigstens 50 Gew.-% Wasser umfasst;
(iii) drei von R¹, R², R³ und R⁴ Wasserstoff darstellen; oder
(iv) einer von R¹, R², R³ und R⁴ -NO₂ darstellt.

10. Verfahren nach Anspruch 1, wobei bezüglich der Herstellung einer Verbindung der Formel II:
(A) das Wasserstoffhalogenid HCl ist; oder
(B) wobei das Lösungsmittelsystem aus Wasser besteht.

11. Verfahren nach einem der vorangehenden Ansprüche, wobei:
(a) die Verbindung der Formel IIA zu der Säure zugegeben wird;
(b) PG¹ =C(R^{q1})OR^{q2} darstellt, wobei R^{q1} und R^{q2} unabhängig C₁₋₆-Alkyl darstellen; und/oder
(c) der Verfahrensschritt weiterhin eine Neutralisation umfasst, um eine freie Base einer Verbindung der Formel II zu erhalten.

12. Verfahren nach einem der Ansprüche 1 bis 11, wobei das Verfahren über ein Zwischenprodukt der Formel XXIV abläuft, 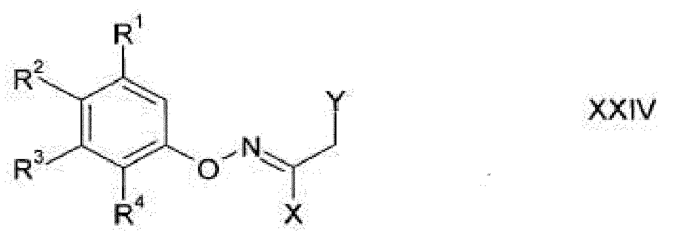 wobei Y, R¹, R², R³, R⁴, X und Z der Definition in einem der Ansprüche 1, 3 oder 4 entsprechen.

13. Verfahren nach einem der Ansprüche 1 bis 12, wobei das Verfahren weiterhin den zusätzlichen Schritt der Kristallisation der Verbindung der Formel I aus einer Lösung umfasst.

14. Verfahren zum Herstellen von:
(I) Dronedaron oder einem Salz davon; oder
(II) einer pharmazeutischen Formulierung, die Dronedaron oder ein Salz davon umfasst, wobei das Verfahren **dadurch gekennzeichnet ist, dass** es als einen Verfahrensschritt ein Verfahren nach einem der Ansprüche 1 bis 13 umfasst.

15. Verfahren zum Herstellen von Dronedaron oder einem Salz davon nach Anspruch 14, das umfasst:
1) ein Verfahren zum Herstellen von 2-Butyl-3-(4-hydroxybenzoyl)-5-nitrobenzofuran oder 2-Butyl-3-(4-methoxybenzoyl)-5-nitrobenzofuran nach einem der Ansprüche 1 bis 14;
2) im Fall von 2-Butyl-3-(4-methoxybenzoyl)-5-nitrobenzofuran), Umwandeln der 4-Methoxy-Komponente in eine 4-Hydroxy-Komponente; gefolgt von, in jeder machbaren Reihenfolge,
3) Umwandeln der Nitro-Gruppe (-NO₂) in eine Methylsulfonylamino-Gruppe (-NHS(O)₂CH₃);
4) Umwandeln der -OH-Gruppe in die -O-(CH₂)₃-N(C₄H₉)₂-Gruppe; und
5) falls nötig/erforderlich, Umwandeln jeder so gebildeten freien Base von Dronedaron in ein Salz.

16. Verfahren nach Anspruch 15, wobei Schritt (1) das Herstellen von 2-Butyl-3-(4-hydroxybenzoyl)-5-nitrobenzofuran umfasst, gefolgt von Schritt (4), dann Schritt (3), dann Schritt (5).

17. Verfahren zum Herstellen einer pharmazeutischen Formulierung, die Dronedaron oder ein Salz davon umfasst, wobei das Verfahren ein Verfahren zum Herstellen von Dronedaron oder einem Salz davon nach Anspruch 14, 15 oder 16 umfasst, gefolgt vom Zusammenbringen des so gebildeten Dronedarons oder eines Salzes davon mit
(i) (einem) pharmazeutisch akzeptablen Hilfsstoff(en), Adjuvans/Adjuvantien, Verdünnungsmittel(n) oder Träger(n); oder
(ii) einem pharmazeutisch unbedenklichen nichtionischen hydrophilen oberflächenaktiven Mittel, ausgewählt aus Poloxameren, und optional einem oder mehreren pharmazeutischen Hilfsstoffen.

18. Verfahren zum Herstellen eines Zwischenprodukts von Dronedaron oder einem Salz davon, wobei das Verfahren einen Verfahrensschritt nach einem der Ansprüche 1 bis 13 umfasst, gefolgt von einem oder mehreren Verfahrensschritten, die in (1), (2), (3) und (4), beschrieben in Anspruch 15, offenbart werden.

## Revendications

1. Procédé de préparation d'un composé de formule I, 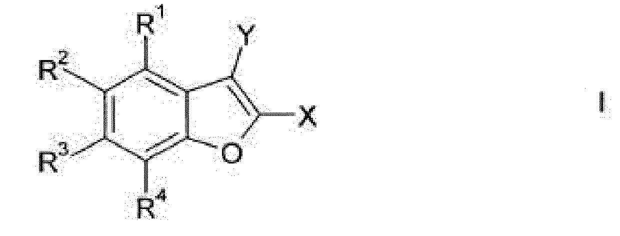 dans lequel R¹, R², R³ et R⁴ représentent indépendamment un hydrogène, un halo, -NO₂, - CN, -C(O)₂R^{x1}, -OR^{x2}, -SR^{x3}, -S(O)R^{x4}, -S(O)₂R^{x5}, -N(R^{x6})R^{x7}, -N(R^{x8})C(O)R^{x9}, -N(R^{x10})S(O)₂R^{x11} ou R^{x12} ;
X représente un hydrogène ou un alkyle en C₁₋₆ facultativement substitué par un ou plusieurs atomes d'halogène ;
Y représente -C(O)-Z ;
Z représente un aryle ou un hétéroaryle, tous deux étant facultativement substitués par un ou plusieurs substituants choisis parmi -OR^{a}, un halo, -NO₂, -CN, -C(O)₂R^{a1}, -SR^{a3}, - S(O)R^{a4}, -S(O)₂R^{a5}, -N(R^{a6})R^{a7}, -N(R^{a8})C(O)R^{a9}, -N(R^{a10})S(O)₂R^{a11} et R^{a12} ;
R^{a} représente un groupe protecteur d'oxygène, un hydrogène ou un alkyle en C₁₋₆ facultativement substitué par un ou plusieurs substituants choisis parmi un halo, -C(O)₂R^{b1} et -N(R^{b2})R^{b3} ;
R^{x1}, R^{x2}, R^{x3}, R^{x6}, R^{x7}, R^{x8}, R^{x9}, R^{x10}, R^{a1}, R^{a3}, R^{a6}, R^{a7}, R^{a8}, R^{a9}, R^{a10}, R^{b1}, R^{a3}, R^{b2} et R^{b3} représentent indépendamment un hydrogène ou un alkyle en C₁₋₆ facultativement substitué par un ou plusieurs atomes d'halogène ;
R^{x4}, R^{x5}, R^{x11}, R^{x12}, R^{a4}, R^{a5}, R^{a11} et R^{a12} représentent indépendamment un alkyle en C₁₋₆ facultativement substitué par un ou plusieurs atomes d'halogène ;
lequel procédé comprend la réaction d'un composé de formule II, 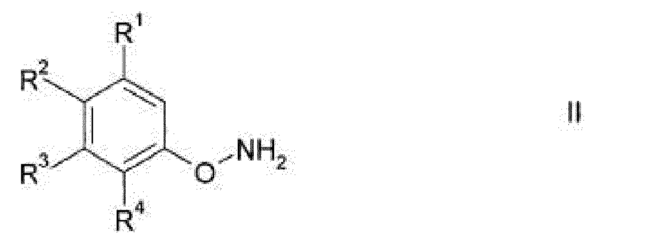 dans lequel :
R¹, R², R³ et R⁴ représentent indépendamment un hydrogène, un halo, -NO₂, -CN, - C(O)₂R^{x1}, -OR^{x2}, -SR^{x3}, -S(O)R^{x4}, -S(O)₂R^{x5}, -N(R^{x6})R^{x7}, -N(R^{x8})C(O)R^{x9}, -N(R^{x10})S(O)₂R^{x11} ou R^{x12} ;
R^{x1}, R^{x2}, R^{x3}, R^{x6}, R^{x7}, R^{x8}, R^{x9}, R^{x10} représentent indépendamment un hydrogène ou un alkyle en C₁₋₆ facultativement substitué par un ou plusieurs atomes d'halogène ;
R^{x4}, R^{x5}, R^{x11} et R^{x12} représentent indépendamment un alkyle en C₁₋₆ facultativement substitué par un ou plusieurs atomes d'halogène ;
avec un composé de formule III, 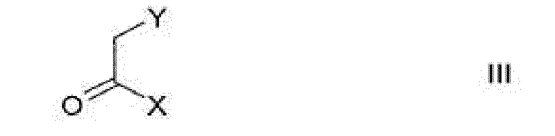
dans lequel Y et X sont tels que définis ci-dessus,
et lequel procédé comprend entre outre la préparation du composé de formule II par le biais d'un procédé comprenant la déprotection d'un composé de formule IIA, 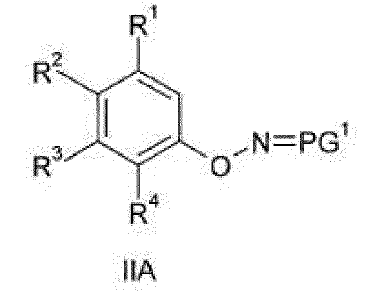
dans lequel :
PG¹ représente un groupe protecteur d'imine ;
et R¹, R², R³ et R⁴ sont tels que définis ci-dessus,
**caractérisé en ce que** la déprotection d'un composé de formule IIA est effectuée en présence d'un halogénure d'hydrogène, d'acide phosphorique ou d'acide sulfurique et d'un système de solvants comprenant au moins 25 % en poids d'eau.

2. Procédé selon la revendication 1, dans lequel le système de solvants est un système dans lequel l'eau est présente dans un rapport molaire de l'eau au solvant organique d'au moins 5:1.

3. Procédé de préparation d'un composé de formule I selon la revendication 1, mais **caractérisé en ce que** :
(a) la réaction est effectuée de façon monotope ;
(b) R² représente -NO₂ ; et/ou
(c) le procédé est effectué en l'absence d'un réactif d'acylation.

4. Procédé selon l'une quelconque des revendications précédentes, dans lequel : X représente du *n*-butyle ; et/ou Z représente du phényle substitué en position *para* par -OH, - OCH₃ ou un -O-benzyle.

5. Procédé selon l'une quelconque des revendications précédentes, dans lequel la réaction utilisée dans la préparation d'un composé de formule I est effectuée en présence d'un acide.

6. Procédé selon la revendication 5, dans lequel l'acide est un acide organique faible.

7. Procédé selon la revendication 6, dans lequel la concentration du composé de formule II dans le solvant acide organique faible se situe entre 0,1 M et 5 M.

8. Procédé selon l'une quelconque des revendications 1 à 7, dans lequel :
(I) le composé de formule II est ajouté au composé de formule III ;
(II) la réaction utilisée dans la préparation d'un composé de formule I est effectuée à température élevée ; et/ou
(III) les composés de formules II et III sont présents dans un rapport molaire compris entre 3:2 et 2:3.

9. Procédé selon la revendication 1, dans lequel en ce qui concerne la préparation d'un composé de formule II :
(i) la réaction est effectuée en présence d'un halogène d'hydrogène ;
(ii) le système de solvants comprend au moins 50 % en poids d'eau ;
(iii) trois parmi R¹, R², R³ et R⁴ représentent un hydrogène ; ou
(iv) l'un quelconque parmi R¹, R², R³ et R⁴ représente -NO₂.

10. Procédé selon la revendication 1, dans lequel en ce qui concerne la préparation d'un composé de formule II :
(A) l'halogénure d'hydrogène est HCl ; ou
(B) le système de solvants est constitué d'eau.

11. Procédé selon l'une quelconque des revendications précédentes, dans lequel :
(a) le composé de formule IIA est ajouté à l'acide ;
(b) PG¹ représente =C(R^{q1})OR^{q2}, où R^{q1} et R^{q2} représentent indépendamment un alkyle en C₁₋₆ ; et/ou
(c) l'étape de procédé comprend en outre la neutralisation pour obtenir une base libre d'un composé de formule II.

12. Procédé selon l'une quelconque des revendications 1 à 11, lequel procédé est effectué par le biais d'un intermédiaire de formule XXIV, 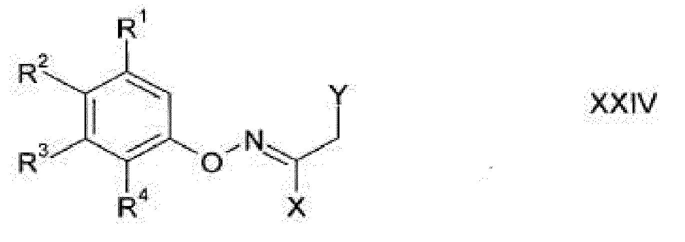 dans lequel Y, R¹, R², R³, R⁴, X et Z sont tels que définis dans l'une quelconque des revendications 1, 3 ou 4.

13. Procédé selon l'une quelconque des revendications 1 à 12, lequel procédé comprend en outre l'étape supplémentaire de cristallisation du composé de formule I à partir d'une solution.

14. Procédé de préparation :
(I) de dronédarone, ou d'un de ses sels ; ou
(II) d'une formulation pharmaceutique comprenant de la dronédarone, ou un de ses sels, lequel procédé est **caractérisé en ce qu'**il comprend une étape de procédé selon l'une quelconque des revendications 1 à 13.

15. Procédé de préparation de dronédarone, ou d'un de ses sels, selon la revendication 14, qui comprend les étapes consistant à :
1) préparer du 2-butyl-3-(4-hydroxybenzoyl)-5-nitrobenzofurane ou du 2-butyl-3-(4-méthoxybenzoyl)-5-nitrobenzofurane selon l'une quelconque des revendications 1 à 14 ;
2) dans le cas de 2-butyl-3-(4-méthoxybenzoyl)-5-nitrobenzofurane, convertir le fragment 4-méthoxy en un fragment 4-hydroxy ; puis, dans un quelconque ordre réalisable,
3) convertir le groupe nitro (-NO₂) en un groupe méthylsulfonylamino (-NHS(O)₂CH₃) ;
4) convertir le groupe -OH en groupe -O-(CH₂)₃-N(C₄H₉)₂ ; et
5) si nécessaire/requis, convertir une quelconque base libre de dronédarone ainsi formée en un sel.

16. Procédé selon la revendication 15, dans lequel l'étape (1) comprend la préparation de 2-butyl-3-(4-hydroxybenzoyl)-5-nitrobenzofurane, suivie de l'étape (4), puis de l'étape (3), puis de l'étape (5).

17. Procédé de préparation d'une formulation pharmaceutique comprenant de la dronédarone, ou un de ses sels, lequel procédé comprend un procédé de préparation de dronédarone, ou d'un de ses sels, selon les revendications 14, 15 ou 16, puis l'association de la dronédarone ou d'un de ses sels ainsi formé, avec
(i) un ou plusieurs excipients, adjuvants, diluants ou vecteurs pharmaceutiquement acceptables ; ou
(ii) un tensioactif hydrophile anionique pharmaceutiquement acceptable choisi parmi les poloxamères et, facultativement, un ou plusieurs excipients pharmaceutiques.

18. Procédé de préparation d'un intermédiaire de dronédarone, ou d'un de ses sels, lequel procédé comprend une étape de procédé selon l'une quelconque des revendications 1 à 13, suivie d'une quelconque ou de plusieurs étapes de procédé décrites en (1), (2), (3) et (4) de la revendication 15.
